# EUROPEAN PATENT APPLICATION

(11) **EP 4 707 388 A1**
(43) Date of publication of application: **11.03.2026**
(21) Application number: 24802747.6
(22) Date of filing: 22.04.2024
(51) Int. Cl.: C12N 9/02, C12N 15/52, C12N 5/10, C12N 15/09, C12P 17/02, C12N 1/15, C12N 1/21, A01H 7/00

(54) **BIOLOGICAL ENZYME THAT CATALYZES FORMATION OF THREE-MEMBERED OR FOUR-MEMBERED OXYGEN-CONTAINING HETEROCYCLIC STRUCTURE OF TAXANE MOLECULE, GENE, BIOLOGICAL MATERIAL, AND USE OF BIOLOGICAL ENZYME, GENE AND BIOLOGICAL MATERIAL**

(30) Priority: 05.05.2023 CN 202310496624
(71) Applicant: Agricultural Genomics Institute at Shenzhen, Chinese Academy of Agricultural Sciences (Shenzhen Branch, Guangdong Laboratory for, Shenzhen, Guangdong 518124 (CN); Agricultural Genomics Institute, Chinese Academy of Agricultural Sciences, Shenzhen, Guangdong 518124 (CN)
(72) Inventor: YAN, Jianbin, Shenzhen, Guangdong 518124 (CN); HUANG, Sanwen, Shenzhen, Guangdong 518124 (CN); JIANG, Bin, Shenzhen, Guangdong 518124 (CN)
(74) Representative: Murgitroyd & Company
(86) International application number: PCT/CN2024/089061
(87) International publication number: WO 2024/230462

(57) **Abstract**

The present invention relates to the technical field of synthetic biology. Provided in the present invention are a biological enzyme that catalyzes the formation of a three-membered or four-membered oxygen-containing heterocyclic structure of a taxane molecule, a gene, a biological material, and the use of the biological enzyme, gene and biological material. The biological enzyme comprises: at least one of amino acid sequences as shown in SEQ ID NOs: 25-48; a fusion protein thereof; a mutant protein thereof; and a homologous protein thereof. The provided biological enzyme can catalyze the formation of a three-membered or four-membered oxygen-containing heterocyclic structure of a taxane molecule, thereby providing new gene resources in terms of increasing the yield of related compounds having an oxetane ring group and an oxirane ring group such as paclitaxel and completing the synthetic pathway thereof; and the nucleotide sequence can be used for modifying a chassis host by means of genetic engineering and metabolic engineering strategies, and producing drugs such as paclitaxel and intermediates thereof, and has significant economic and social values.

## Description

The application relates to the field of synthetic biology technology, and particularly to a bioenzyme for catalyzing formation of three-membered or four-membered oxygen heterocyclic structures of a taxane molecule, and gene, biomaterial and use thereof.

### BACKGROUND

Taxanes are a series of derivatives synthesized by separating active anti-tumor ingredients from plants and modifying the structures of the compounds of the active anti-tumor ingredients. Taxane drugs mainly include paclitaxel, docetaxel, cabazitaxel, and derivatives with taxane skeleton structure.

Paclitaxel is a diterpenoid alkaloid with a structural formula as shown below:

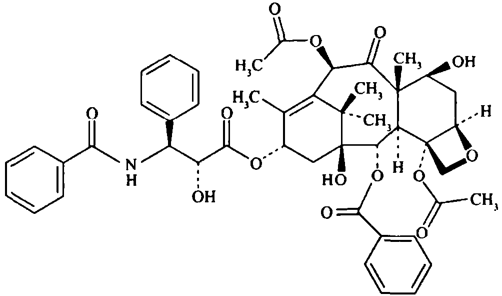

Paclitaxel was first isolated from the bark of *Taxus brevifolia,* and currently it is widely used in the clinical treatment of various cancers.

Currently, the acquisition of paclitaxel active pharmaceutical ingredient (API) mainly relies on three approaches, including plant extraction, chemical/semi-chemical synthesis, and biosynthesis, but none of them can meet market demand. Semi-chemical synthesis is to first extract the natural precursor substances baccatin III and 10-deacetylbaccatin III (10-DAB) from leaves, then chemically synthesizing paclitaxel. However, the precursor substances used in this method still rely on plant extraction and are restricted by plant resources, and cannot completely solve the supply problem. In the field of commercial production, cell culture has already been used to produce paclitaxel active pharmaceutical ingredient. However, the initial paclitaxel production from bark cells is too low. In addition, due to insufficient understanding of the paclitaxel synthesis pathway, it is difficult to significantly increase the paclitaxel yield by conventional metabolic engineering strategies. Therefore, by analyzing the key enzymes in paclitaxel synthesis, and then using synthetic biology and metabolic engineering strategies to reconstruct the paclitaxel synthesis pathway in plants or microbial chassis, mass production of paclitaxel by cell culture is expected to completely solve the problem of paclitaxel supply and demand.

As a tension four-membered heterocyclic ring, oxetane can serve as both a stable group in a biologically active compound and a reactive synthetic intermediate. Oxetane is frequently used in drug development due to its "drug-like" property as a replacement group for gem-dimethyl and carbonyl with improved physicochemical properties. The enormous diversity and complexity of natural oxyheterocyclobutane compounds (OCCs) in structure provide unique advantages and opportunities for modem drug discovery and development. More than 600 different OCCs have been found in animals, plants, and microorganisms, with the majority produced by plants. Three reaction mechanisms have been discovered for the biosynthesis of natural oxetane skeletons: [2+2] cycloaddition, ring contraction, and ring expansion. [2+2] cycloaddition and ring contraction mechanisms have been discovered in animals and microorganisms. In contrast, ring expansion reaction currently exists only in plants. However, the enzymes for catalyzing the ring-expansion reaction and oxetane formation are unknown.

The largest number of OCCs found in plants is in the *Taxaceae* family, and the best-known example is paclitaxel. Paclitaxel has a complex 6-8-6 tricyclic carbon skeleton with 9 stereocenters, a distinct oxetane ring group, and a phenylisoserine chain. Since the structure of paclitaxel was first reported in 1971, how the oxetane ring is formed in *Taxus chinensis* has attracted extensive attention from synthetic and computational chemists. The biosynthesis of paclitaxel goes through 19 steps, including hydroxylation, acetylation, oxidation and epoxidation, where the epoxidation is the most critical step, and the related enzymes have not yet been reported.

Accordingly, the discovery and identification of the taxane C4-C20 oxetane-forming enzymes is a crucial step in improving the entire paclitaxel synthesis pathway, and is also necessary for the future heterologous reconstruction of the paclitaxel synthesis pathway.

### SUMMARY

In view of this, the application provides a bioenzyme for catalyzing the formation of three-membered or four-membered oxygen heterocyclic structure of taxane molecule, nucleic acid molecule, biomaterial and use thereof. The bioenzyme can be used to synthesize oxygen-containing heterocyclic compounds (such as paclitaxel and an intermediate thereof).

In order to achieve the above object of the application, the application provides the following technical solutions:
The application provides a bioenzyme including at least one of the following:
a1) at least one of the amino acid sequences represented by SEQ ID NOs: 25-48;
a2) a fusion protein obtained by connecting a tag to N-terminus and/or C-terminus of at least one of the amino acid sequences represented by SEQ ID NOs: 25-48;
a3) a protein having the same function obtained by substituting and/or deleting and/or adding one or more amino acid residues in at least one of the amino acid sequences represented by SEQ ID NOs: 25-48;
a4) a protein having at least 60% homology with at least one of the amino acid sequences represented by SEQ ID NOs: 25-48 and having the same function.

The inventors of the application screened and isolated a taxane C4-C20 oxetane-forming enzyme gene from *Taxus chinensis var. maire* through large-scale resource screening, which is named TOT in the application, and successfully obtained the encoded protein of the gene (i.e., taxane C4-C20 oxetane-forming enzyme, also named TOT in the application). Functional analysis revealed that TOT can catalyze the substrate taxa-4(20),11-diene-2α,5α,7β,9α,10β,13α-hexaol-hexa-acetate (taxadiene-hexaol-hexaacetate, compound (1)) or a derivative thereof to produce two products, of which 1-dehydroxybaccatin IV (compound (2)) is the main product, and the other product is baccatin I (compound (3)). The taxane C4-C20 oxetane-forming enzyme gene TOT provides genetic resources for the production of 1-dehydroxybaccatin IV and baccatin I.

This application creatively identifies a taxane C4-C20 oxetane forming enzyme from *Taxus chinensis.* The taxane C4-C20 oxetane forming enzyme TOT provides a synthetic pathway for the production of 1-dehydroxybaccatin IV and baccatin I.

Further, the application obtains a homologous protein and a mutant of TOT, proving that the homologous protein and mutant can also catalyze the production of 1-dehydroxybaccatin IV and baccatin I.

Further, the catalytic reaction technology can guide the synthesis of a compound having an oxetane ring group and/or a compound having an oxirane ring group.

In a particular embodiment of this application, in the fusion protein obtained by connecting a tag to N-terminus and/or C-terminus of the amino acid sequences represented by SEQ ID NO: 25-48, the connecting tag is not particularly limited and can be added according to the intended use, for example, it may be selected from but not limited to: HIS, GST, Flag, MBP, HA, c-Myc, eGFP, eYFP, and eCFP.

In an embodiment of the application, the protein in a3) is a protein obtained by adding, deleting, and/or substituting one or more (for example, 1, 2, 3, 4, 5, 6, 7, 8, 9, 10 or more) amino acid residues in the amino acid sequence of a1) and having the same bioenzyme activity.

In an embodiment of the application, the protein in a3) is a protein obtained by adding, deleting, and/or substituting one or more (for example, 1, 2, 3, 4, 5, 6, 7, 8, 9, 10 or more) amino acid residues in the amino acid sequence of a1) and having similar or better bioenzyme activity.

In a particular embodiment of the application, the protein in a3) is a mutant of the amino acid sequence of a1), and the mutant(s) include at least one of the following mutants: V237A, P167A, Q187A, Q6A, V129A, I172A, S314A, V319A, Q381A, E313A, V205A, R50A, S500A, P428A, H249A, S310A, K259A, R269A, M177A, D361A, V382A, I347A, V371A, G448A, P454AA, P490A, I285A, T410A, T231A, K360A, G455A, or K94A. The above amino acids are represented in the form of single letter abbreviations, and the Chinese name, English name, and English abbreviation of the amino acids are the common names in this field.

In an embodiment of the application, the protein in a4) is a protein having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% homology to the amino acid sequence of a1) and having the same function.

Optionally, the amino acid sequence of a4) is derived from the genus *Taxus,* including but not limited to: *Taxus baccata L., Taxus brevifolia* Nutt., *Taxus calcicola* L.M. Gao & Mich. Möller, *Taxus canadensis* Marshall, *Taxus chinensis* (Pilg.) Rehd., *Taxus contorta* Griff., *Taxus cuspidata* Siebold & Zucc., *Taxus Florida* Nutt ex Chapm., *Taxus florini* Spjut, *Taxus globosa* Schltdl., *Taxus mairei* (Lemée & Lév.) S.Y. Hu, *Taxus phytoni* Spjut, *Taxus sumatrana* (Miq.) de Laub, *Taxus wallichiana* Zucc., *Taxus* Huangshan type, *Taxus* Emei type, *Taxus* Qinling type, *Taxus yunnanensis* W. C. Cheng&L. K. Fu, *Taxus* × *media* Rehder, *Taxus* × *hunnewelliana* Rehder, and others.

In a particular embodiment of the application, the homologous sequence of the amino acid sequence represented by SEQ ID NO: 25 is the amino acid sequence represented by SEQ ID NOs: 26-48. The homology between the homologous sequences represented by SEQ ID NOs: 26-48 and the amino acid sequence represented by SEQ ID NO: 25 is 64.8%-100%.

In a particular embodiment of this application, the above bioenzyme is an isolated bioenzyme.

The application further provides a gene encoding the above bioenzyme, which include at least one of the following sequences:
b1) at least one of the nucleotide sequences represented by SEQ ID NOs: 1-24;
b2) a complementary sequence, a degenerate sequence or a homologous sequence of at least one of the nucleotide sequences represented by SEQ ID NOs: 1-24, where the homologous sequence is a nucleotide sequence having at least 75% homology with the nucleotide sequence represented by SEQ ID NOs: 1-24;
b3) a nucleotide sequence hybridizing with the nucleotide sequence of b1) or b2) under stringent conditions and being capable of encoding a protein with the same function.

In an embodiment of the application, the gene is a nucleic acid molecule.

Optionally, the nucleic acid molecule is a synthetic and/or isolated nucleic acid molecule.

In an embodiment of the application, the complementary sequence is a complementary sequence formed according to the principle of complementary base pairing, and the complementary sequence may be an incomplete complementary sequence or a complete complementary sequence having the same function as the nucleotide sequence of b1).

In an embodiment of the application, a degenerate sequence means that after changing one or more nucleotides in the nucleotide sequence of b1), the type of amino acid encoded by the changed nucleotide sequence position remains unchanged, and the function and expression level of the gene will not be affected.

In an embodiment of the application, a homologous sequence is a nucleotide sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% homology with the nucleotide sequence of b1) and having the same function; for example, a mutant gene, an allele, or a derivative.

Optionally, the nucleotide sequence of the nucleic acid molecule of b2) is derived from the genus *Taxus,* including but not limited to: *Taxus baccata L., Taxus brevifolia* Nutt., *Taxus calcicola* L.M. Gao & Mich. Möller, *Taxus canadensis* Marshall, *Taxus chinensis* (Pilg.) Rehd., *Taxus contorta* Griff., *Taxus cuspidata* Siebold & Zucc., *Taxus Florida* Nutt ex Chapm., *Taxus florini* Spjut, *Taxus globosa* Schltdl., *Taxus mairei* (Lemée&Lév.) S.Y. Hu, *Taxus phytoni* Spjut, *Taxus sumatrana* (Miq.) de Laub, *Taxus wallichiana* Zucc., *Taxus* Huangshan type, *Taxus* Emei type, *Taxus* Qinling type, *Taxus yunnanensis W.* C. Cheng&L. K. Fu, *Taxus* × *media* Rehder, *Taxus* × *hunnewelliana* Rehder, and others.

Illustratively, "stringent conditions" refer to conditions under which a probe will hybridize to its target sequence to a detectable degree exceeding hybridization to other sequences (e.g., at least 2-fold over background). Stringent conditions are sequence-dependent, and may be different in different circumstances. By controlling the stringency of the hybridization and/or washing conditions, target sequences that are 100% complementary to a probe can be identified. Alternatively, stringency conditions can be adjusted to allow some sequence mismatching so that lower degrees of similarity are detected.

In a particular embodiment of the application, the homologous sequence of the nucleotide sequence represented by SEQ ID NO: 1 is the nucleotide sequences represented by SEQ ID NOs: 2-24. The homology between the homologous sequences represented by SEQ ID NOs: 2-24 and the nucleotide sequence represented by SEQ ID NO: 1 is 77.9%-100%.

This application provides a biomaterial, which is any one of the following c1) to c4):
c1) an expression cassette including the above gene;
c2) a recombinant vector including the above gene or the expression cassette of c1);
c3) a recombinant microorganism including at least one of: the above gene, the expression cassette of c1), or the recombinant vector of c2); and
c4) a recombinant plant cell and/or recombinant animal cell including at least one of: the above gene, the expression cassette of c1), the recombinant vector of c2), or the recombinant microorganism of c3).

In an embodiment of the application, the recombinant plant cell and/or recombinant animal cell include a non-biologically transformed (produced by a non-substantial biological method) recombinant plant cell and/or recombinant animal cell.

In an embodiment of the application, the expression cassette of c1) further includes a promoter operatively connected to the above nucleic acid molecule. Optionally, the expression cassette of c1) further includes other regulatory elements, such as enhancer, leader sequence, transposon, terminator, marker gene, etc.

In an embodiment of the application, in the recombinant vector of c2), the type of the vector is not particularly limited, and a suitable vector can be selected according to practical needs. For example, the vectors include but are not limited to: pFastBac1, pYES2, pYES2.1, pESC-Ura, pESC-Trp, pESC-Leu, pESC-His, pGEX2T, pTAex3, pUSA, pYMB0, pHT43, pET28b, pIJ702, pUCP19, pYMB03 or pHT43; preferably pESC-Ura.

In an embodiment of the application, in the recombinant microorganism of c3), the microbial chassis includes but is not limited to at least one of: *Streptomyces, Pseudomonas, Bacillus,* yeast cell, or *Escherichia coli.*

In the embodiments of the application, the microorganisms of c3) include bacteria and/or fungi.

In an embodiment of the application, the bacteria include but are not limited to: *Escherichia* cells, *Lactobacillus* cells, *Lactococcus* cells, *Corynebacterium* bacteria, *Acetobacter* bacteria, *Acinetobacter* bacteria, *Pseudomonas* cells, *Streptomyces* cells, *Bacillus* cells, *Staphylococcus* cells, *Agrobacterium* cells, and endophytes of *Taxus.* For example, *Escherichia coli, Bacillus subtilis, Agrobacterium tumefaciens, Agrobacterium rhizogenes, Lactococcus lactis, Bacillus cereus, Pseudomonas fluorescens,* etc. In some embodiments, bacterial cells such as *Escherichia spp., Streptomyces spp., Zymonas spp., Acetobacter spp., Citrobacter spp., Synechocystis spp., Rhizobium spp., Clostridium spp., Corynebacterium spp., Streptococcus spp., Xanthomonas spp., Lactobacillus spp., Lactococcus spp., Bacillus spp., Alcaligenes spp., Pseudomonas spp., Aeromonas spp., Azotobacter spp. spp., Comamonas spp., Mycobacterium spp., Rhodococcus spp., Gluconobacter spp., Ralstonia spp., Acidithiobacillus spp., Microlunatus spp., Geobacter spp., Geobacillus spp., Arthrobacter spp., Flavobacterium spp., Serratia spp., Saccharopolyspora spp., Thermus spp., Stenotrophomonas spp., Chromobacterium spp, Sinorhizobium spp, Saccharopolyspora spp, Agrobacterium spp,* and *Pantoea spp.* The bacterial cells may be Gram-negative cells, such as *E. coli* cells, or Gram-positive cells, such as *Bacillus* species.

In an embodiment of the application, the fungus includes but is not limited to: yeast, filamentous fungi or mushrooms.

In a particular embodiment of the application, yeast includes but is not limited to: *Saccharomyces cerevisiae, Schizosaccharomyces pombe, Yarrowia lipolytica, Candida utilis, Pichia pastoris, Kluyveromyces lactis, Hansenula polymorpha, Candida boidinii, Adenysera adenilyticus, Phaffia rhodozyma, and Candida albicans.* The yeast cells are, for example, *Saccharomyces spp., Schizosaccharomyces spp., Pichia spp., Paffia spp., Kluyveromyces spp., Candida spp., Talaromyces spp., Brettanomyces spp., Pachysolen spp., Debaryomyces spp., Yarrowia spp.* and industrial polyploid yeast strains. Preferably, the yeast strain is a S. *cerevisiae* strain or a *Yarrowia* strain.

In a particular embodiment of the application, the filamentous fungi include but are not limited to: *Monascus, Aspergillus oryzae, Aspergillus niger, Aspergillus flavus, and Penicillium.* The fungi are, for example, *Aspergillus spp., Penicillium spp., Fusarium spp., Rhizopus spp., Acremonium spp., Neurospora spp., Sordaria spp., Magnaporthe spp., Allomyces spp., Ustilago spp., Botrytis spp.* and *Trichoderma spp.*

This application further provides an engineered strain including at least one of the above nucleic acid molecule, expression cassette, or vector. In an embodiment of the application, the engineering strain may be a yeast engineering strain, or an *Escherichia coli* engineering strain.

The application further provides a yeast engineering strain, which includes at least one of the above gene, expression cassette or recombinant vector.

In a particular embodiment of the application, the recombinant microorganism of c3) is *Saccharomyces cerevisiae* including the pESC-Ura::TOT plasmid. TOT in the pESC-Ura::TOT plasmid refers to the nucleotide sequences of b1) to b3).

In an embodiment of the application, in the recombinant plant cell of c4), the plant cell chassis includes but is not limited to at least one of: *Taxus chinensis* cells, tobacco cells, *Pseudotaxus chienii* cells, *Artemisia annua* cells, *Arabidopsis* cells, *Physcomitrella patens* cells, *Marchantia polyphylla* cells, or algae cells.

In the embodiments of the application, the plant cells of c4) include but are not limited to: tobacco cells, *Pseudotaxus chienii* cells, *Artemisia annua* cells, *Arabidopsis* cells, *Physcomitrella patens* cells, *Marchantia polyphylla* cells, tomato cells, ginseng cells, cotton cells, sugarcane cells, potato cells, corn cells, wheat cells, rice cells, radish cells, lettuce cells, algae cells, etc., and the plant cells include protoplasts, suspension cells, etc.

In the embodiments of the application, the algal cells include but are not limited to at least one of: blue-green algae (cyanobacteria), green algae, *Synechococcus,* slender *Synechococcus, Synechocystis, Anabaena, Chlamydomonas,* and *Chlamydomonas reinhardtii.*

In an embodiment of the application, the recombinant animal cell of c4), the animal cell chassis includes but is not limited to: insect cells, mammalian cells, *drosophila* cells, nematode cells, and fish cells.

In the embodiments of the application, the animal cells of c4) include but are not limited to at least one of: insect cells, mammalian cells, nematode cells, and fish cells.

In a particular embodiment of the application, insect cell includes but is not limited to: S2 *Drosophila* cells or Sf9 cell.

In a particular embodiment of the application, mammalian cell includes but are not limited to: fibroblast, lymphocyte, epithelial cell, and myeloblast. For example, HEK293 cell, CHO cell, COS cell, BHK cell, HeLa cell, Chinese hamster ovary cell, Vero cell, SP2/0 cell, NS/0 myeloma cell, hamster kidney cell, human B cell, human T cell Jurkat, neuronal cell, CV-I/EBNA cell, L cell, 3T3 cell, HEPG2 cell, and MDCK cell.

In a particular embodiment of the application, fish cell includes but is not limited to zebrafish cell.

In some embodiments of the application, the recombinant plant cell or recombinant animal cell expresses the bioenzyme and produces a target product by cell culture, which does not include developing the recombinant cell or recombinant animal cell into a complete plant or animal.

Among the above biomaterials, the methods for introducing a gene, expression cassette, and recombinant vector into a host cell in vivo and in vitro include but are not limited to: electroporation, polyethylene glycol (PEG) transformation, lipofection, heat shock, calcium phosphate precipitation, virus mediation, and microinjection. Host cells include microorganisms, plant cells and/or animal cells.

The application further provides a method for producing a host cell, where the method includes transforming the host cell with at least one of the above nucleic acid molecules, expression cassettes or vectors in biomaterials.

In the embodiments of the application, the host cell includes microbial cell, plant cell, animal cell, and/or algal cell.

The application further provides a method for producing producing a plants or plant cell, the method includes transforming the plant or plant cell with at least one of the above nucleic acid molecules, expression cassettes or vectors in biomaterials.

The application provides a method for expressing the above bioenzyme, where at least one of the TOT gene of the application, or an expression cassette thereof, or a recombinant vector thereof, is transferred into microbial cells to allow the microbial cells to express the bioenzyme.

The application provides another method for expressing the above bioenzyme, where at least one of the TOT gene of the application, or an expression cassette thereof, or a recombinant vector thereof, and a recombinant microorganism is transferred into plant cells or animal cells, so that the plant cells or animal cells express the bioenzyme.

This application provides a method for producing the above bioenzyme, where the method includes: transforming a host cell with at least one of the above nucleic acid molecule, or the expression cassette or vector in the biomaterial, so that the host cell produces the bioenzyme.

In the embodiments of the application, the host cell includes microbial cell, plant cell, animal cell, and/or algal cell.

The application provides use of any one of the above bioenzyme, gene or biomaterial in the synthesis of an oxygen-containing heterocyclic compound, where the oxygen-containing heterocyclic compound includes a compound having an oxetane ring group and/or a compound having an oxirane ring group.

In an embodiment of the application, the compound having an oxetane ring group includes a taxane compound, and/or an intermediate thereof;
preferably, the taxane compound includes but is not limited to at least one of: paclitaxel and a derivative thereof, docetaxel and a derivative thereof, or cabazitaxel and a derivative thereof;
preferably, the taxane compound intermediate includes but is not limited to at least one of: 1-dehydroxybaccatin IV and a derivative thereof, baccatin III and a derivative thereof, or 10-deacetylbaccatin III and a derivative thereof.

In an embodiment of the application, the compound having oxirane ring group includes at least one of: baccatin I and a derivative thereof, Taxumairol C and a derivative thereof, Taxuchin A and a derivative thereof.

The application provides a method for synthesizing an oxygen-containing heterocyclic compounds in vivo or in vitro, where a taxane compound including 5α-acetoxy-4,20-alkenyl or a taxane compound including 5α-hydroxy-4,20-alkenyl is used as a substrate to synthesize the oxygen-containing heterocyclic compound under the catalytic action of the above bioenzyme;
the oxygen-containing heterocyclic compound includes: a compound having an oxetane ring group, and/or a compound having an oxirane ring group.

In an embodiment of the application, the substrate includes but is not limited to at least one of: taxadiene-hexol-hexaacetate, taxadiene-2α,5α,7β,9β,10β,13α-hexol-5α-acetate, 5α-acetate-Taxuspine F, taxadiene-hexol-tetraacetate, or Taxuspine F.

In an embodiment of the application, the in vivo includes in a microorganism, in a plant cell and/or in an animal cell.

In an embodiment of the application, the in vivo includes in an algal cell.

In an embodiment of the application, synthesizing the oxygen-containing heterocyclic compound in vivo includes: transferring at least one of the above gene, expression cassette, recombinant vector, and recombinant microorganism into a plant cell or an animal cell, so that the plant cell or animal cell expresses the bioenzyme of the application; and using a taxane compound including 5α-acetoxy-4,20-alkenyl or a taxane compound including 5α-hydroxy-4,20-alkenyl as a substrate to synthesize the oxygen-containing heterocyclic compound under the catalytic action of the bioenzyme.

In an embodiment of the application, synthesizing the oxygen-containing heterocyclic compound in vivo includes: transferring at least one of the above nucleic acid molecule, or expression cassette or vector in the biomaterial into a host cell to produce the bioenzyme; and using a taxane compound including 5α-acetoxy-4,20-alkenyl or a taxane compound including 5α-hydroxy-4,20-alkenyl as a substrate to synthesize the oxygen-containing heterocyclic compound under the catalytic action of the bioenzyme.

Optionally, the substrate is produced by the microbial cell, algae cell, plant cell, and/or animal cell.

Optionally, the substrate can be artificially added in addition.

This application further provides a plant or a plant part thereof, where the plant is one of the following plants:
(dl) a transgenic plant including the above nucleic acid molecule;
(d2) a plant formed by growth of the above plant cell;
(d3) a plant produced by the above method;
(d4) an offspring formed by self-pollination of any plant of (d1) to (d3), as well as a plant formed by growth of the offspring;
(d5) an offspring formed by hybridization of any plant of (d1) to (d3) with other varieties, as well as a plant formed by growth of the offspring; and
where the above plant part includes a root, stem, leaf, flower, fruit, pollen or seed.

The application further provides a method for manufacturing a commercial product, the method includes: obtaining the above plant or plant part thereof, and manufacturing the commercial product from the plant or plant part thereof; where the commercial product is a crude extract, an active pharmaceutical ingredient, and/or a pharmaceutical formulation including at least one selected from the group consisting of: baccatin III and a derivative thereof, paclitaxel and a derivative thereof, taxusin and a derivative thereof, docetaxel and a derivative thereof, and cabazitaxel and a derivative thereof.

Compared with the prior art, the application has the following beneficial effects:
(1) The application provides a taxane C4-C20 oxetane-forming enzyme TOT, and identifies a novel, key biosynthetic enzyme that can catalyze the oxidation of carbon-carbon double bond of a taxane molecule to form an oxetane and an oxirane, thereby significantly enhancing the progress of the analysis of the biosynthetic pathways of paclitaxel and other related compounds having an oxetane ring group or an oxirane ring group, and also greatly reducing the difficulty of completely analyzing the synthetic pathways of compounds such as paclitaxel. This lays a solid foundation for producing paclitaxel through cell culture and for reducing the production costs of compound drugs such as paclitaxel. It is expected to effectively solve the current situation of high prices and insufficient supply of paclitaxel drugs on the market.
(2) The nucleotide sequence encoding a taxane C4-C20 oxetane-forming enzyme TOT provided by the application provides a new gene resource for increasing the yield of paclitaxel and other related compounds having an oxetane ring group or an oxirane ring group, and completing their synthetic pathway. The nucleotide sequence can be used for genetic engineering and metabolic engineering strategies to transform a chassis host and produce paclitaxel and other drugs and their intermediates, etc., which has significant economic and social value.

### BRIEF DESCRIPTION OF THE DRAWINGS

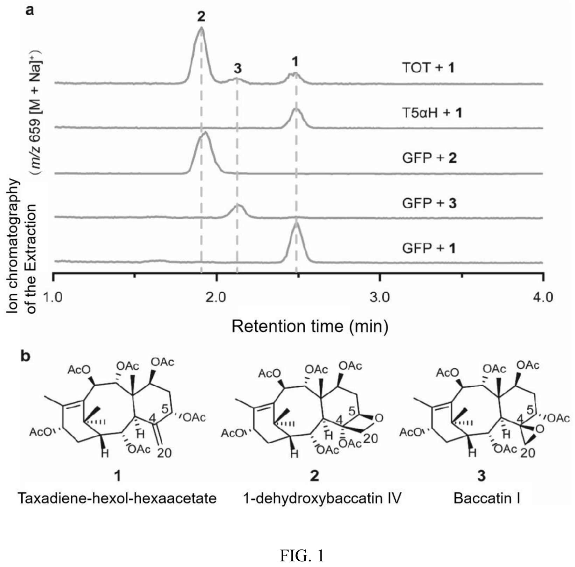

Fig. 1 shows the results of characterizing the activity of TOT1 in tobacco;
   particularly, a of Fig. 1 shows the results of the extracted ion chromatography (EIC) for transiently expressing TOT in tobacco and catalyzing compound (1), as compared with the control, TOT can transform compound (1) into two new products, compound (2) and compound (3);
   b of Fig. 1 shows the structural formulas of the three compounds: standard compound (1), compound (2), and compound (3).
Figs. 2a-2b show the activity comparison results of key amino acid mutations of the taxane C4-C20 oxetane-forming enzyme TOT;
   particularly, Fig. 2a shows the difference fold of compound (2) produced by transforming compound (1) by different mutants as compared with the wild type (TOT^{WT});
Fig. 2b shows the difference fold of compound (3) produced by transforming compound (1) by different mutants as compared with TOT^{WT}, and mutants with a difference fold between the upper and lower dashed lines are considered to have no significant difference from the wild type;
Fig. 3 shows the results of the extracted ion chromatography (EIC) for yeast expressing TOT and catalyzing compound (1), as compared with the control, the taxane C4-C20 oxetane-forming enzyme TOT can transform compound (1) into two new products, compound (2) and compound (3).
Fig. 4 shows the results of the extracted ion chromatography (EIC) for insect cells expressing TOT and catalyzing compound (1), as compared with the control, the taxane C4-C20 oxetane-forming enzyme TOT can transform compound (1) into two new products, compound (2) and compound (3).
Fig. 5 shows the activity difference between different homologous genes of the taxane C4-C20 oxetane-forming enzyme TOT;
   particularly, a of Fig. 5 shows the difference fold in the content of compound (2) produced by transforming compound (1) with other homologous genes, as compared with TOT1;
   b of Fig. 5 shows the fold difference of compound (3) produced by transforming compound (1) with other homologous genes, as compared with TOT1.

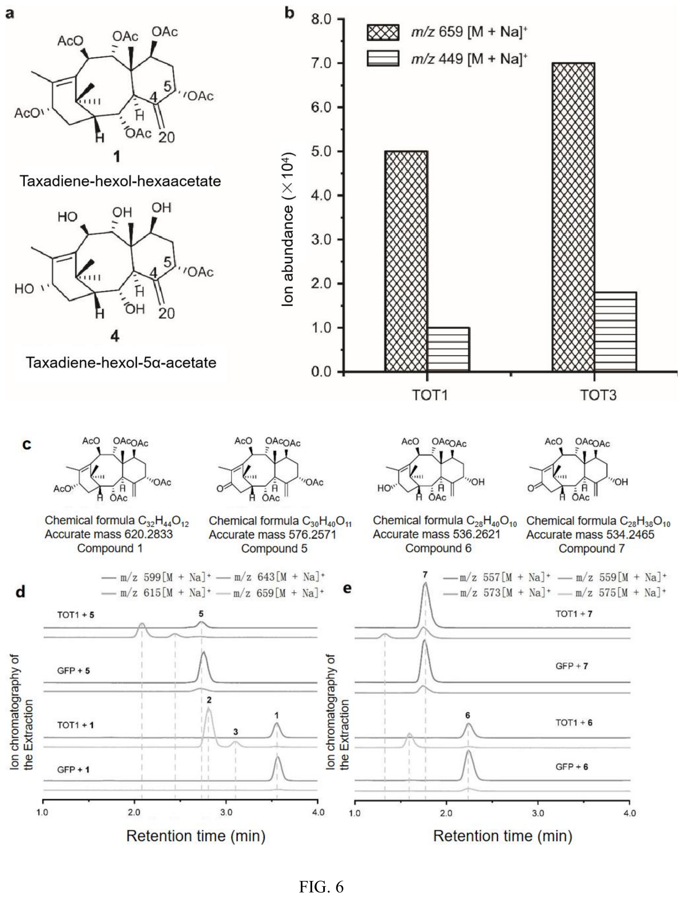

Fig. 6 shows the comparison of the transformation efficiency of different homologous genes of the taxane C4-C20 oxetane-forming enzyme TOT on different compounds, and the oxidation activity of TOT1 on compound (1), compound (5), compound (6) and compound (7);
   particularly, a of Fig. 6 shows the structural formulas of the two compounds used, compound (1) and compound (4);
   b of Fig. 6 shows the content of oxidation products produced by catalyzing compound (1) and compound (4) with TOT1 and TOT3 respectively, which is expressed as ion abundance;
   c of Fig. 6 shows the structural formulas of compound (1), compound (5), compound (6) and compound (7);
   d of Fig. 6 shows the results of the extracted ion chromatogram for two oxidation products produced by respectively catalyzing compound (1) and compound (5) with TOT; and
   e of Fig. 6 shows the results of the extracted ion chromatogram for the oxidation products produced by respectively catalyzing compound (6) and compound (7) with TOT.

### DETAILED DESCRIPTION OF THE EMBODIMENTS

The application discloses a bioenzyme for catalyzing formation of three-membered or four-membered oxygen heterocyclic structures of a taxane molecule, and nucleic acid molecule, biomaterial and use thereof. Those skilled in the art can refer to the content of this application and appropriately improve the process parameters to achieve it. It should be particularly noted that, all similar substitutions and modifications are obvious to those skilled in the art, and they are deemed to be included in the application. The method and use according to the application have been described through preferred embodiments. Relevant personnel can obviously modify or appropriately modify and combine the method and use described herein without departing from the content, spirit and scope of the application to implement and apply the technology of the application.

### Terminology Explanation:

In the application, the term "TOT" may refer to a taxane C4-C20 oxetane forming enzyme, or a taxane C4-C20 oxetane forming enzyme gene, or a nucleotide sequence encoding a taxane C4-C20 oxetane forming enzyme, and the specific meaning may be determined in combination with the context.

In the application, the term "bioenzyme" or "enzyme" refers to a protein (or "polypeptide" or "peptide composition") with biological activity, including both naturally occurring proteins and variants and modified forms thereof. As used herein, the terms "protein" and "polypeptide" are used interchangeably, and thus the term polypeptide can be used to refer to a full-length polypeptide as well as a fragment of a full-length polypeptide. The term "fragment" refers to a portion of a polypeptide sequence. A "fragment" or "biologically active portion" includes a polypeptide including a sufficient number of contiguous amino acid residues to retain biological activity, such as a polypeptide in which the N-terminal amino acid has been truncated.

"Variant" means a substantially similar sequence. As will be readily appreciated by those skilled in the art, naturally occurring proteins may have some differences between different species or between different samples, which may be deletion and/or addition of one or more amino acids at one or more internal sites and/or substitution of one or more amino acids at one or more sites of the native polypeptide. However, this does not affect their ability to play the same or similar roles, and these proteins can be referred to as natural variants of the exemplary proteins. Modifications of proteins include, but are not limited to, appropriate amino acid substitutions/additions/deletions, truncation of N-terminal amino acid, codon optimization suitable for host cell preferences, tag addition and fusion, etc., which do not affect the biological activity of the target protein. These proteins may be referred to as artificial variants of the reference proteins. Guidance for appropriate amino acid substitutions that do not affect the biological activity of the target protein can refer to the model described in Dayhoff et al. (1978) Atlas of Protein Sequence and Structure (Natl. Biomed. Res. Found., Washington,D.C.), which is incorporated herein by reference. Conservative substitutions, such as replacing one amino acid with another having similar properties, may be optimal.

In the application, the term "amino acid" refers to any amino acid (both standard and non-standard amino acids), including but not limited to: α-amino acid, β-amino acid, γ-amino acid and δ-amino acid. Examples of suitable amino acids include, but are not limited to, alanine, asparagine, aspartic acid, cysteine, glutamic acid, glutamine, glycine, proline, serine, tyrosine, arginine, histidine, isoleucine, leucine, lysine, methionine, phenylalanine, threonine, tryptophan, and valine.

In the application, the term "taxane C4-C20 oxetanase" refers to an enzyme that catalyzes the oxidation of carbon-carbon double bonds of taxane molecule into oxetane and oxirane. The term "TOT" may refer to taxane C4-C20 oxetanase, or taxane C4-C20 oxetanase gene, or a nucleotide molecule or sequence encoding taxane C4-C20 oxetanase, and the specific meaning may be determined in combination with the context.

In some embodiments, the bioenzymes related to the application can be isolated from a material including a given bioenzyme from any source. Any method of obtaining the bioenzymes related to the application is compatible with the application.

As used herein, the term "isolating" means removing from its natural environment or from other compound present when the compound is first formed. The term "isolated" includes a material separated from a natural source, as well as a material recovered following production by recombinant expression in a host cell (such as nucleic acids and proteins), or a chemically synthesized compound (such as a nucleic acid molecule, protein and peptide).

In the application, the term "nucleic acid molecule" (or "nucleic acid" or "polynucleotide") may refer to a polymeric form of nucleotides, which may include sense and antisense strands of RNA, cDNA, genomic DNA, as well as synthetic forms and mixed polymers thereof. A nucleotide may refer to ribonucleotide, deoxyribonucleotide, or modified form of either type of nucleotide. As used herein, "nucleic acid molecule" is synonymous with "nucleic acid" and "polynucleotide." A nucleic acid molecule is usually at least 10 bases in length unless otherwise specified. The term can refer to an RNA or DNA molecule of indeterminate length. The term includes both single-stranded and double-stranded forms of DNA. Nucleic acid molecules can include one or both of naturally occurring and modified nucleotides linked together by naturally occurring and/or non-naturally occurring nucleotide linkages.

As will be readily appreciated by those skilled in the art, for nucleotide sequences, well-known molecular biology techniques, such as the polymerase chain reaction (PCR) and hybridization techniques outlined herein, can be used to identify naturally occurring variants, i.e., substantially similar sequences.

As will be readily appreciated by those skilled in the art, nucleic acid molecules may be chemically or biochemically modified, or may include non-natural or derived nucleotide bases. Such modifications include, for example, labeling, methylation, substitution of one or more naturally occurring nucleotides with an analog, internucleotide modifications (e.g., uncharged bonds: e.g., methylphosphonate, phosphotriester, phosphoramidite, carbamate, etc.; charged bonds: e.g., phosphorothioate, phosphorodithioate, etc.; pendant moieties: e.g., peptide; intercalator: e.g., acridine, psoralen, etc.; chelating agents; alkylating agents; and modified bonds: e.g., α-anomeric nucleic acids, etc.). The term "nucleic acid molecule" further includes any topological conformation, including single-stranded, double-stranded, partially double-stranded, triple-stranded, hairpin, circular, and padlocked conformations.

In some embodiments, the nucleic acid molecules related to the application may be cloned from DNA including a given nucleic acid molecule from any source, for example, by PCR amplification and/or restriction enzyme digestion. In some embodiments, the nucleic acid molecules related to this application are synthetic. Any method of obtaining nucleic acid molecules related to the invention is compatible with the application.

In the application, the term "identity" refers to sequence similarity with natural nucleic acid sequences or amino acid sequences. Identity can be assessed by the naked eye or by computer software. Using computer software, the identity between two or more sequences can be expressed as a percentage (%), which can be used to evaluate the identity between related sequences.

Unless otherwise indicated, the sequence identity values provided herein refer to the values obtained using the full-length sequences of the application by Jalview version 2.11.2.7 (Waterhouse, A.M., Procter, J.B., Martin, D.M.A, Clamp, M. and Barton, G.J. (2009) "Jalview Version 2 -- a multiple sequence alignment editor and analysis workbench" Bioinformatics 25(9) 1189-1191 doi:10.1093/bioinformatics/btp033), and using the default parameters in the multiple alignment software package MUSCLE version v3.8.31 ("MUSCLE: multiple sequence alignment with high accuracy and high throughput" Nucleic Acids Res. 32(5):1792 (2004)); or any equivalent program thereof).

In this application, the term "homology" is sometimes used to refer to the level of similarity between two or more nucleic acid sequences or amino acid sequences expressed as a percentage of positional identity (i.e., sequence similarity or identity). Homology also refers to the concept of evolutionary correlation, usually demonstrated by similar functional characteristics between different nucleic acids or proteins that share similar sequences.

As easily understood by technical personnel in this field, homologous sequences can be obtained by aligning known sequences with genome or transcriptome data of species samples with similar evolutionary relationships. For example, between different species of the same genus or between different plants of the same species, the homologous sequence of the sequence can be obtained by aligning the known sequence in the sample genome or transcriptome data. Those skilled in the art can expect it to have the same or similar function.

Additional mathematical algorithms are known in the art and can be used to align two sequences. See, for example, the algorithms in Karlin and Altschul (1990) Proc. Natl. Acad. Sci. USA 87:2264, such as a modification in Altschul (1993) Proc. Natl. Acad. Sci. USA 90:5873-5877. This algorithm was introduced in the BLAST program of Altschul et al. (1990) J.Mol. Biol. 215:403. BLAST nucleotide searches can be performed with the BLASTN program (nucleotide query for nucleotide sequence searches) to obtain nucleotide sequences homologous to the nucleic acid molecules of the application, or with the BLASTX program (translated nucleotide query for protein sequence searches) to obtain protein sequences homologous to the nucleic acid molecules of the application. BLAST protein searches can be performed with the BLASTP program (protein query for protein sequence searches) to obtain amino acid sequences homologous to protein molecules of the application, or with the TBLASTN program (protein query for translated nucleotide sequence searches) to obtain nucleotide sequences homologous to protein molecules of the application. To obtain gapped alignments for comparison purposes, Gapped BLAST (in BLAST 2.0) can be used as described in Altschul et al. (1997) Nucleic Acids Res. 25:3389. Alternatively, PSI-Blast can be used to perform an iterated search that detects distant relationships between molecules. See Altschul et al. (1997) supra. When utilizing BLAST, Gapped BLAST, and PSI-Blast programs, the default parameters of the respective programs (e.g., BLASTX and BLASTN) can be used. Alignment can also be performed manually by inspection.

In this application, the term "synthetic" refers to polynucleotide (i.e., DNA or RNA) molecules produced by chemical synthesis as an in vitro process. For example, synthetic DNA can be produced in a reaction process within an Eppendorf^{™} tube, such that synthetic DNA is enzymatically produced from a natural DNA or RNA strand. Other laboratory methods may be utilized to synthesize polynucleotide sequences. Oligonucleotides can be chemically synthesized on an oligonucleotide synthesizer by solid phase synthesis using phosphoramidites. Synthetic oligonucleotides can anneal to each other as a complex, thereby producing a "synthetic" polynucleotide. Other methods for chemically synthesizing polynucleotides are known in the art, and can be readily adapted for use with the present disclosure.

In this application, the term "gene" is defined as a genetic unit (usually represented by a DNA sequence) that occupies a specific position in the chromosome and includes genetic instructions that contribute to potential phenotypic features or traits of the plant. In some scenarios, the term "gene" refers to a nucleic acid molecule expressing a specific protein. A "gene" includes DNA regions that encode a gene product, as well as all DNA regions that regulate the production of a gene product, whether or not such regulatory sequences are adjacent to the coding and/or transcribed sequences. Thus, genes include, but are not necessarily limited to: promoter sequences, terminators, translation regulatory sequences, such as ribosome binding sites and internal ribosome entry sites, enhancers, silencers, insulators, boundary elements, replication origins, matrix attachment sites, introns, and locus control regions.

In the application, the term "gene product" refers to any product generated by a gene. For example, a gene product can be a direct transcription product of the gene (such as mRNA, tRNA, rRNA, antisense RNA, interfering RNA, ribozyme, structural RNA, or any other type of RNA), or it can be a protein produced by translation of mRNA.

In the application, the term "expression cassette" refers to a DNA fragment that can be inserted into a nucleic acid or polynucleotide at a specific restriction site or by homologous recombination. As used herein, a DNA fragment includes a polynucleotide encoding a polypeptide of interest, and an expression cassette and restriction sites are designed to ensure insertion of the expression cassette into the proper reading frame for transcription and translation. In one embodiment, an expression cassette may include a polynucleotide encoding a polypeptide of interest and, in addition to the polynucleotide, have elements that facilitate transformation of a particular host cell. In one embodiment, the expression cassette may further include elements that allow for enhanced expression of the polynucleotide encoding the polypeptide of interest in the host cell. These elements may include, but are not limited to, promoters, minimal promoters, enhancers, response elements, terminator sequences, polyadenylation sequences, and the like.

In the application, the term "vector" is used interchangeably with "construct", "cloning vector" and "expression vector", and means a vector that can introduce DNA or RNA sequences (such as foreign genes) into host cells to transform the host and promote the expression (such as transcription and translation) of the introduced sequences. "Non-viral vector" refers to any vector that does not include a virus or retrovirus. In some embodiments, a "vector" is a DNA sequence including at least one DNA replication origin and at least one selectable marker gene. Examples include, but are not limited to, plasmids, cosmids, bacteriophages, bacterial artificial chromosomes (BACs), or viruses that carry foreign DNA into cells. The vector may further include one or more genes, antisense molecules and/or selectable marker genes and other genetic elements known in the art. The vector may transduce, transform or infect cells, thereby causing the cells to express the nucleic acid molecules and/or proteins encoded by the vector.

**In** the application, the term "expression" refers to the biosynthesis of a gene product, including the transcription and/or translation of the gene product. "Expressing" or "producing" a protein or polypeptide from a DNA molecule refers to transcribing and translating the coding sequence to produce the protein or polypeptide, while "expressing" or "producing" a protein or polypeptide from an RNA molecule refers to translating the RNA coding sequence to produce the protein or polypeptide.

Expression vectors including all necessary expression elements are commercially available, and familiar to those skilled in the art. See, e.g., Sambrook et al., Molecular Cloning: A Laboratory Manual, 2nd ed., Cold Spring Harbor Laboratory Press, 1989. Cells are genetically engineered by introducing exogenous DNA (RNA) into the cells. The exogenous DNA (RNA) is placed under the effective control of transcription elements to allow the exogenous DNA to be expressed in the host cell.

In the application, the term "transformation" includes all techniques by which a nucleic acid molecule can be introduced into such a cell. Examples include, but are not limited to: transfection with viral vectors; transformation with plasmid vectors; electroporation; fat infection; microinjection; *Agrobacterium-mediated* transfer; direct DNA uptake; WHISKERS^{™} mediated transformation; and microprojectile bombardment. These techniques can be used for both stable and transient transformation of host cells. "Stable transformation" refers to the introduction of a nucleic acid fragment into the genome of a host organism, resulting in genetic stability. Once stably transformed, the nucleic acid fragment is stably integrated into the genome of the host organism and any subsequent generations. Host organisms including the transformed nucleic acid fragments are referred to as "transgenic" organisms. "Transient transformation" refers to the introduction of a nucleic acid fragment into the nucleus or DNA-including organelle of a host organism, resulting in gene expression without genetically stable inheritance.

In some embodiments, to transform hosts and host cells, the nucleotide sequence of the application can be inserted into any vector known in the art suitable for expressing nucleotide sequences in hosts or host cells by using standard techniques. The choice of vector depends upon the preferred transformation technique and the target host species to be transformed. The transformation method depends on the host cell to be transformed, the stability of the vector used, the expression level of the gene product and other parameters.

In the application, the "stringent conditions" mentioned above may be any one of: low stringency conditions, medium stringency conditions, and high stringency conditions. "Low stringency conditions" include, for example, the conditions of: 5×SSC, 5×Denhardt's solution, 0.5% SDS, 50% formamide, and 32°C. "Moderately stringent conditions" include, for example, the conditions of: 5×SSC, 5×Denhardt's solution, 0.5% SDS, 50% formamide, and 42°C. "High stringent conditions" include, for example, the conditions of: 5×SSC, 5×Denhardt's solution, 0.5% SDS, 50% formamide, and 50°C. Among the above conditions, the higher the temperature, the more efficiently a DNA having high homology can be expected to be obtained. Factors that affect hybridization stringency include: temperature, probe concentration, probe length, ionic strength, time, salt concentration, and other factors. Those skilled in the art can achieve the same stringent conditions by appropriately selecting these factors.

In the above description of the figures and the following Examples, the full names of the compounds are as follows:

**Table 1: Full names of Compounds (1) to (7)**

| Compounds | Full chemical names of the compounds |
|---|---|
| Compound (1) | Taxa-4(20),11-diene-2α,5α,7β,9α,10β,13α-hexaol- hexa-acetate |
| Compound (2) | 1-dehydroxybaccatin IV |
| Compound (3) | Baccatin I |
| Compound (4) | Taxa-4(20),11-diene-2α,5α,7β,9α,10β,13α-hexaol- 5α-acetate |
| Compound (5) | 5α-acetate-taxuspine F |
| Compound (6) | Taxa-4(20),11-diene-2α,5α,7β,9α,10β,13α-hexaol- 2α,7β,9α,10β-acetate |
| Compound (7) | Taxuspine F |

**Table 2: The similarity of an amino acid sequence to a TOT bioenzyme**

| Name of bioenzyme | SEQ ID NO: | Similarity to TOT1 | Source |
|---|---|---|---|
| TOT1 | 25 | 100% | *Taxus chinensis var. maire* |
| TOT2 | 26 | 94.08% | *Taxus chinensis var. maire* |
| TOT3 | 27 | 96.45% | *Taxus chinensis var. maire* |
| TOT4 | 28 | 95.46% | *Taxus chinensis var. maire* |
| TOT5 | 29 | 94.78% | *Taxus chinensis var. maire* |
| TOT6 | 30 | 93.69% | *Taxus chinensis var. maire* |
| TOT7 | 31 | 93.89% | *Taxus chinensis var. maire* |
| TOT8 | 32 | 94.38% | *Taxus chinensis var. maire* |
| TOT9 | 33 | 94.18% | *Taxus chinensis var. maire* |
| TOT10 | 34 | 93.89% | *Taxus chinensis var. maire* |
| TOT11 | 35 | 94.38% | *Taxus chinensis var. maire* |
| TOT12 | 36 | 96.59% | *Taxus chinensis var. maire* |
| TOT13 | 37 | 93.78% | *Taxus chinensis var. maire* |
| TOT14 | 38 | 97.44% | *Taxus baccata L.* |
| TOT15 | 39 | 95.07% | *Taxus baccata L.* |
| TOT16 | 40 | 97.39% | *Taxus baccata L.* |
| TOT17 | 41 | 94.98% | *Taxus baccata L.* |
| TOT18 | 42 | 94.28% | *Taxus cuspidata* Siebold & Zucc. |
| TOT19 | 43 | 67.47% | *Taxus chinensis* (Pilg.) Rehd. |
| TOT20 | 44 | 94.18% | *Taxus cuspidata* Siebold & Zucc. |
| TOT21 | 45 | 97.44% | *Taxus wallichiana* Zucc. |
| TOT22 | 46 | 97.39% | *Taxus wallichiana* Zucc. |
| TOT23 | 47 | 93.49% | *Taxus wallichiana* Zucc. |
| TOT24 | 48 | 63.64% | *Taxus chinensis var. maire* |

**Table 3: The similarity of a nucleotide sequence to a TOT bioenzyme**

| Name of bioenzyme | SEQ ID NO: | Similarity to TOT1 |
|---|---|---|
| TOT1 | 1 | 100% |
| TOT2 | 2 | 96.21% |
| TOT3 | 3 | 97.84% |
| TOT4 | 4 | 97.06% |
| TOT5 | 5 | 96.73% |
| TOT6 | 6 | 96.14% |
| TOT7 | 7 | 96.14% |
| TOT8 | 8 | 96.73% |
| TOT9 | 9 | 96.60% |
| TOT10 | 10 | 96.08% |
| TOT11 | 11 | 96.46% |
| TOT12 | 12 | 97.87% |
| TOT13 | 13 | 96.01% |
| TOT14 | 14 | 98.10% |
| TOT15 | 15 | 96.66% |
| TOT16 | 16 | 98.07% |
| TOT17 | 17 | 96.60% |
| TOT18 | 18 | 96.14% |
| TOT19 | 19 | 77.56% |
| TOT20 | 20 | 96.07% |
| TOT21 | 21 | 98.30% |
| TOT22 | 22 | 98.27% |
| TOT23 | 23 | 96.08% |
| TOT24 | 24 | 74.85% |

The reagents, instruments, strains or other biomaterials used in the application are commercially available.

The application will be further described below in conjunction with the Examples:

### Example 1: Expression of Taxane C4-C20 Oxetane Forming Enzyme TOT Gene in Tobacco

### 1. cDNA Acquisition

*Taxus chinensis* var. *maire* was preserved in the laboratory. After collecting the young leaves, they were immediately quick-frozen in liquid nitrogen. Some samples were taken to grind in a mortar. 100 mg of ground sample was added to a 1.5 mL centrifuge tube, and lysis buffer was added to extract RNA (Plant Total RNA Isolation Kit Plus, FOREGENE, China). The mRNA was reverse transcribed into cDNA by using a reverse transcription kit (HiScript III 1st Strand cDNA Synthesis Kit, Vazyme, China).

### 2. Cloning of Taxane C4-C20 Oxetane-forming Enzyme TOT

The genome of *Taxus chinensis var. mairei* was analyzed to obtain 83 genes of the CYP725A subfamily of *Taxus chinensis var. mairei.* The reading frame nucleotide sequence and amino acid sequence of the TOT1 are respectively represented by SEQ ID NO: 1 (its homologous sequence is represented by SEQ ID NOs: 2-24 in the sequence listing) and SEQ ID NO: 25 (its homologous sequence is represented by SEQ ID NOs: 26-48 in the sequence listing). The names of the enzymes corresponding to homologous sequences are TOT2 to TOT24

The sequence of SEQ ID NO: 1 is as follows:

ATGGTTCATGTGTTGCAGGTAGTGAAAATGGATAGAGTTAGAGAAATATTTAATGGAAGTTCA
GGTTCTCCAGCTGGTATTCCCCACAGTGTGATCACAGCCGGTGTGGGTGCCATAATAATAATTCTTCTGT
CACTACTGCTCCTCCGCCGTTCTAGTAAACGGGGCGACTCCTCTCATCCTCCTGGGAATTCAGGCCTTCC
ATTCATTGGAGAGACATTATCATTCACCAAGGCTTTTAAATCGAACACGCTGGCCGAATTTTTTGAGGAG
AGGGTGAAGAAATTCGGGAATGTATTTAAGATTTCAATAATCGGGCCTCCCACAGTGGTAATGTGCGGC
AATGAGGGAAACCGGTTTATTTTCGCCAACGAGGAGAAGCTTGTCCACTTGTCGTGGTCTGGTCGATAT
GCGAAAATCCTGGGTGGAGATTCCGTTTCCATGAAGAGGGGAGATGATCATCGCAGTGTACGTGCCGCA
TTCGCAGGGTTTTTGAGCCCTGCATCGCTGCCTATTTACATAAGTAAAATGAGTGCACAGATCCAAGATC
ATATCAACCAAAAATGGAAAGGAAAAGATGTAATTGCTGTAGTTCCTCTGGTAAAGGAGCTCGTCTTCA
ACGTTTCCTACAACTTGTTTTTCAGCATAAATGATAGCGAGGAACTGCATCGATTGCATAAGATTTTCGA
AACTATTGTGGAGGGACATGTTTCCATGCCGATAGACCTTCCCGGATTCACCTTTCATAGAGCACTTCAG
GGAAGGGCGAAGCTCAAGAAAGTTTTCTCTTCTTTAATAGAAAGGAGAAGAAGCGATCTGAGCTCCGG

ATTGGCATCTGCTAATCAGGATCTCATCTCTGTTTTACTCACCTACAAAGATGATAGGGGGTATACAATGA
CCCACGACGAGCTCCTCGACAACTTTCTTTCCCTTCTTGAATCCTCCTATGATTCCGTCAATTCACCAATG
GCCTGCATTTTTAAGCTTTTGTATGCCAATCCAGAATGCTATGAAAAAGTAGTTCAAGAGCAATTGGGGA
TACTTTCTGGTAAGAAGGAAGGACAAGAAATCTCGTGGAAGGATCTGAGATCCATGAAATACACATGGC
AAGTACTTCAGGAAACGCTACGACTGTATACTCAAGTTGCTGGAATATTTCGCAAAGCCATGACTGTCAT
TCATTATGATGGTCACACCATTCCCAAAGGGTGGCAACTTCTTTGGGCAACCCAAACTACACATCTGAA
CGACAAATATTTCAGTGAGCCGGAAAAATTCATGCCTTCCAGATTCGATGAAGAAGGAAACAATGTGAT
TCCTTACTCATTCGTACCATTTGGAGGAGGGCGGCGGATGTGTCCAGGTTGGGAATTCGGAAAGATGGA
GATCTTACTCTTCGTCCATCACTTTGTTAAAGCGTTCAGTGGCTTTACCCCAATCGATCCGGACGAAAAA
ATTACTGGGAATCCATTTCCTCATCTCCCTGCCAATGGATTTTCAATAAAACCTACTCCCAGATCCTAA.

The sequence of SEQ ID NO: 25 is as follows:

MVHVLQVVKMDRVREIFNGSSGSPAGIPHSVITAGVGAIIIILLSLLLLRRSSKRGDSSHPPGNSGLP
FIGETLSFTKAFKSNTLAEFFEERVKKFGNVFKISIIGPPTVVMCGNEGNRFIFANEEKLVHLSWSGRYAKILG
GDSVSMKRGDDHRSVRAAFAGFLSPASLPIYISKMSAQIQDHINQKWKGKDVIAVVPLVKELVFNVSYNLFF
SINDSEELHRLHKIFETIVEGHVSMPIDLPGFTFHRALQGRAKLKKVFSSLIERRRSDLSSGLASANQDLISVL
LTYKDDRGYTMTHDELLDNFLSLLESSYDSVNSPMACIFKLLYANPECYEKVVQEQLGILSGKKEGQEISW
KDLRSMKYTWQVLQETLRLYTQVAGIFRKAMTVIHYDGHTIPKGWQLLWATQTTHLNDKYFSEPEKFMPS
RFDEEGNNVIPYSFVPFGGGRRMCPGWEFGKMEILLFVHHFVKAFSGFTPIDPDEKITGNPFPHLPANGFSIK
PTPRS.

Based on the nucleotide sequence of the TOT gene, primers P1 and P2 for amplifying the TOT gene were designed. The primers included a portion of the sequence of the tobacco expression vector pEAQ-HT. The TOT gene was amplified by PCR using the cDNA of *Taxus chinensis var. mairei* leaves as a template. The PCR target product was recovered by gel excision, and then recombined with the linear pEAQ-HT vector digested with RruI and XhoI. The ClonExpress one-step cloning kit (Vazyme Biotech) was used for cloning and sequencing, and the positive recombinant plasmid was named pEAQ-HT-TOT.

Among the primers used, P1 and P2 were used to amplify TOT, and the primer sequences are shown in Table 4.

**Table 4: Primer sequences**

| Name | Sequence | SEQ ID NO: |
|---|---|---|
| P1 | 5'-gtatattctgcccaaattcgcgaATGGTTCATGTGTTGCAGG-3' | 49 |
| P2 | 5'-accagagttaaaggcctcgagTTAGGATCTGGGAGTAGGTTTTATTGAAAAT C-3' | 50 |
| P3 | 5'-gtcaaggagaaaaaaccccggatccATGGTTCATGTGTTGCAGG-3' | 51 |
| P4 | 5'-aaatcaacttctgttccatgtcgacGGATCTGGGAGTAGGTTTTATTG-3' | 52 |
| P5 | 5'-ttcaaaggcctacgtcgacgagctcATGGTTCATGTGTTGCAGGTAGTGAAAAT -3' | 53 |
| P6 | 5'-caggctctagattcgaaagcggccgcctagtgatggtgatggtgatgGGATCTGGGAGTAG GTTTTATTGAAAATCCAT-3' | 54 |
| P7 | 5'-ttcaaaggcctacgtcgacgagctcATGCAGGCTAATTCCAACACGGT-3' | 55 |
| P8 | 5'-caggctctagattcgaaagcggccgctcagtgatggtgatggtgatgCCATATATCTCGTAA GTATCTTCCATCCATCTGT-3' | 56 |
| P9 | 5'-gtatattctgcccaaattcgcgaATGGTTCATGTGTTGCAGGT-3' | 57 |
| P10 | 5'-accagagttaaaggcctcgagTTAGGATCTGAGAATAGGTTTTATTAGAAAT CC-3' | 58 |
| P11 | 5'-gtatattctgcccaaattcgcgaATGGTTCATGTGTTGCACG-3' | 59 |
| P12 | 5'-accagagttaaaggcctcgagTTAGGATCTGAGAATAGGTTTTATTAGAAAT CCATTG-3' | 60 |
| P13 | 5'-gtatattctgcccaaattcgcgaATGGTTCATGTGTTGCAGGT-3' | 61 |
| P14: | 5'-accagagttaaaggcctcgagTTAGGATCTGAGAATAGGTTTTATTAGAAAT CCATTG-3' | 62 |
| Q6A-F | TCATGTGTTGGCTGTAGTGAAAATGGATAGAGTTAGAGAAATATTT AATGGAAGTTCAG | 63 |
| Q6A-R | TTTTCACTACAGCCAACACATGAACCATtcgcgaatttg | 64 |
| F122A-F | CCGGTTTATTGCTGCCAACGAGGAGAAGCTTGTCC | 65 |
| F122A-R | CCTCGTTGGCAGCAATAAACCGGTTTCCCTCATTGCC | 66 |
| V129A-F | GGAGAAGCTTGCTCACTTGTCGTGGTCTGGTCGATATG | 67 |
| V129A-R | ACGACAAGTGAGCAAGCTTCTCCTCGTTGGCGAAAATAAAC | 68 |
| L131A-F | GCTTGTCCACGCTTCGTGGTCTGGTCGATATGCG | 69 |
| L131A-R | CAGACCACGAAGCGTGGACAAGCTTCTCCTCGTTGG | 70 |
| W133A-F | CCACTTGTCGGCTTCTGGTCGATATGCGAAAATCCTGG | 71 |
| W133A-R | ATCGACCAGAAGCCGACAAGTGGACAAGCTTCTCC | 72 |
| Y137A-F | GTCTGGTCGAGCTGCGAAAATCCTGGGTGGAGATTC | 73 |
| Y137A-R | GGATTTTCGCAGCTCGACCAGACCACGACAAGTG | 74 |
| D144A-F | CCTGGGTGGAGCTTCCGTTTCCATGAAGAGGGGAGATG | 75 |
| D144A-R | TGGAAACGGAAGCTCCACCCAGGATTTTCGCATATCGAC | 76 |
| V146A-F | TGGAGATTCCGCTTCCATGAAGAGGGGAGATGATCATCG | 77 |
| V146A-R | TCTTCATGGAAGCGGAATCTCCACCCAGGATTTTCGC | 78 |
| S147A-F | AGATTCCGTTGCTATGAAGAGGGGAGATGATCATCGC | 79 |
| S147A-R | CCCTCTTCATAGCAACGGAATCTCCACCCAGG | 80 |
| H154A-F | GGGAGATGATGCTCGCAGTGTACGTGCCG | 81 |
| H154A-R | GTACACTGCGAGCATCATCTCCCCTCTTCATGGAAACG | 82 |
| R158A-F | TCGCAGTGTAGCTGCCGCATTCGCAGGGT | 83 |
| R158A-R | CGAATGCGGCAGCTACACTGCGATGATCATCTCCCCTC | 84 |
| P167A-F | GTTTTTGAGCGCTGCATCGCTGCCTATTTACATAAGTAAAATGAGTG | 85 |
| P167A-R | GCAGCGATGCAGCGCTCAAAAACCCTGCGAATGCG | 86 |
| I172A-F | ATCGCTGCCTGCTTACATAAGTAAAATGAGTGCACAGATCCAAGAT CATATC | 87 |
| I172A-R | TACTTATGTAAGCAGGCAGCGATGCAGGGC | 88 |
| Q187A-F | TCATATCAACGCTAAATGGAAAGGAAAAGATGTAATTGCTGTAGTT CC | 89 |
| Q187A-R | CTTTCCATTTAGCGTTGATATGATCTTGGATCTGTGCACTCATTTTAC | 90 |
| V237A-F | GGAGGGACATGCTTCCATGCCGATAGACCTTCCCG | 91 |
| V237A-R | TCGGCATGGAAGCATGTCCCTCCACAATAGTTTCGAAAATCTTATG | 92 |
| S310A-F | CAACTTTCTTGCTCTTCTTGAATCCTCCTATGATTCCGTCAAT | 93 |
| S310A-R | ATTCAAGAAGAGCAAGAAAGTTGTCGAGGAGCTCGT | 94 |
| E313A-F | TTCCCTTCTTGCTTCCTCCTATGATTCCGTCAATTCACC | 95 |
| E313A-R | CATAGGAGGAAGCAAGAAGGGAAAGAAAGTTGTCGAGGAG | 96 |
| S314A-F | CCTTCTTGAAGCTTCCTATGATTCCGTCAATTCACCAATGGC | 97 |
| S314A-R | AATCATAGGAAGCTTCAAGAAGGGAAAGAAAGTTGTCGAGG | 98 |
| S318A-F | CTCCTATGATGCTGTCAATTCACCAATGGCCTGCAT | 99 |
| S318A-R | GTGAATTGACAGCATCATAGGAGGATTCAAGAAGGGAAAGAAAG | 100 |
| V319A-F | CTATGATTCCGCTAATTCACCAATGGCCTGCATTTTTAAGC | 101 |
| V319A-R | TTGGTGAATTAGCGGAATCATAGGAGGATTCAAGAAGGGAAAG | 102 |
| L330A-F | TTTTAAGCTTGCTTATGCCAATCCAGAATGCTATGAAAAAGTAG | 103 |
| L330A-R | GATTGGCATAAGCAAGCTTAAAAATGCAGGCCATTGG | 104 |
| I347A-F | GCAATTGGGGGCTCTTTCTGGTAAGAAGGAAGGACAAGAAATCT | 105 |
| I347A-R | TACCAGAAAGAGCCCCCAATTGCTCTTGAACTACTTTTTCAT | 106 |
| D361A-F | CTCGTGGAAGGCTCTGAGATCCATGAAATACACATGGCAAG | 107 |
| D361A-R | TGGATCTCAGAGCCTTCCACGAGATTTCTTGTCCTTCCT | 108 |
| M365A-F | TCTGAGATCCGCTAAATACACATGGCAAGTACTTCAGGAAACG | 109 |
| M365A-R | ATGTGTATTTAGCGGATCTCAGATCCTTCCACGAGATTTC | 110 |
| Y367A-F | ATCCATGAAAGCTACATGGCAAGTACTTCAGGAAACG | 111 |
| Y367A-R | CTTGCCATGTAGCTTTCATGGATCTCAGATCCTTCCACG | 112 |
| V371A-F | CACATGGCAAGCTCTTCAGGAAACGCTACGACTGTATACT | 113 |
| V371A-R | TTTCCTGAAGAGCTTGCCATGTGTATTTCATGGATCTCAGATC | 114 |
| R377A-F | GGAAACGCTAGCTCTGTATACTCAAGTTGCTGGAATATTTCGC | 115 |
| R377A-R | GAGTATACAGAGCTAGCGTTTCCTGAAGTACTTGCCATG | 116 |
| Q381A-F | ACTGTATACTGCTGTTGCTGGAATATTTCGCAAAGCC | 117 |
| Q381A-R | TTCCAGCAACAGCAGTATACAGTCGTAGCGTTTCCTGAAG | 118 |
| V382A-F | GTATACTCAAGCTGCTGGAATATTTCGCAAAGCCATG | 119 |
| V382A-R | ATATTCCAGCAGCTTGAGTATACAGTCGTAGCGTTTCCTG | 120 |
| G384A-F | TCAAGTTGCTGCTATATTTCGCAAAGCCATGACTGTCAT | 121 |
| G384A-R | TGCGAAATATAGCAGCAACTTGAGTATACAGTCGTAGCG | 122 |
| I385A-F | AGTTGCTGGAGCTTTTCGCAAAGCCATGACTGTCATTC | 123 |
| I385A-R | CTTTGCGAAAAGCTCCAGCAACTTGAGTATACAGTCGTAGC | 124 |
| R387A-F | TGGAATATTTGCTAAAGCCATGACTGTCATTCATTATGATGG | 125 |
| R387A-R | TCATGGCTTTAGCAAATATTCCAGCAACTTGAGTATACAGTCGT | 126 |
| I400A-F | TGGTCACACCGCTCCCAAAGGGTGGCAACTTCT | 127 |
| I400A-R | ACCCTTTGGGAGCGGTGTGACCATCATAATGAATGACAGTCAT | 128 |
| W404A-F | TCCCAAAGGGGCTCAACTTCTTTGGGCAACCCAAACT | 129 |
| W404A-R | AAAGAAGTTGAGCCCCTTTGGGAATGGTGTGACC | 130 |
| T410A-F | TCTTTGGGCAGCTCAAACTACACATCTGAACGACAAATATTTCAGT G | 131 |
| T410A-R | GTGTAGTTTGAGCTGCCCAAAGAAGTTGCCACCC | 132 |
| F426A-F | GCCGGAAAAAGCTATGCCTTCCAGATTCGATGAAGAAGG | 133 |
| F426A-R | TGGAAGGCATAGCTTTTTCCGGCTCACTGAAATATTTGTCG | 134 |
| P428A-F | AAAATTCATGGCTTCCAGATTCGATGAAGAAGGAAACAATGTG | 135 |
| P428A-R | CGAATCTGGAAGCCATGAATTTTTCCGGCTCACTGAAATATTTG | 136 |
| F443A-F | TCCTTACTCAGCTGTACCATTTGGAGGAGGGCGG | 137 |
| F443A-R | CAAATGGTACAGCTGAGTAAGGAATCACATTGTTTCCTTCTTCATC | 138 |
| G455A-F | GATGTGTCCAGCTTGGGAATTCGGAAAGATGGAGATCTTACT | 139 |
| G455A-R | CGAATTCCCAAGCTGGACACATCCGCCGCC | 140 |
| P445A-F | CTCATTCGTAGCTTTTGGAGGAGGGCGGCG | 141 |
| P445A-R | CTCCTCCAAAAGCTACGAATGAGTAAGGAATCACATTGTTTCCTTC | 142 |
| F446A-F | ATTCGTACCAGCTGGAGGAGGGCGGCGGAT | 143 |
| F446A-R | GCCCTCCTCCAGCTGGTACGAATGAGTAAGGAATCACATTGTTTCC TTCTTC | 144 |
| G447A-F | CGTACCATTTGCTGGAGGGCGGCGGATGTG | 145 |
| G447A-R | GCCGCCCTCCAGCAAATGGTACGAATGAGTAAGGAATCACATTGTT TCCTTCT | 146 |
| G448A-F | ACCATTTGGAGCTGGGCGGCGGATGTGTC | 147 |
| G448A-R | TCCGCCGCCCAGCTCCAAATGGTACGAATGAGTAAGGAATCACAT | 148 |
| R450A-F | TGGAGGAGGGGCTCGGATGTGTCCAGGTTGGG | 149 |
| R450A-R | GACACATCCGAGCCCCTCCTCCAAATGGTACGAATGAG | 150 |
| R451A-F | AGGAGGGCGGGCTATGTGTCCAGGTTGGGAATTCGG | 151 |
| R451A-R | CTGGACACATAGCCCGCCCTCCTCCAAATGGTAC | 152 |
| M452A-F | AGGGCGGCGGGCTTGTCCAGGTTGGGAATTCGGAAAG | 153 |
| M452A-R | AACCTGGACAAGCCCGCCGCCCTCCTCC | 154 |
| P454A-F | GCGGATGTGTGCTGGTTGGGAATTCGGAAAGATGGAGAT | 155 |
| P454A-R | ATTCCCAACCAGCACACATCCGCCGCCC | 156 |
| P490A-F | TACTGGGAATGCTTTTCCTCATCTCCCTGCCAATGG | 157 |
| P490A-R | GATGAGGAAAAGCATTCCCAGTAATTTTTTCGTCCGGATC | 158 |
| F491A-F | TGGGAATCCAGCTCCTCATCTCCCTGCCAATGG | 159 |
| F491A-R | GGAGATGAGGAGCTGGATTCCCAGTAATTTTTTCGTCCGG | 160 |
| P492A-F | GAATCCATTTGCTCATCTCCCTGCCAATGGATTTTCAAT | 161 |
| P492A-R | CAGGGAGATGAGCAAATGGATTCCCAGTAATTTTTTCGTCCG | 162 |
| S500A-F | CAATGGATTTGCTATAAAACCTACTCCCAGATCCTAActcgag | 163 |
| S500A-R | TAGGTTTTATAGCAAATCCATTGGCAGGGAGATGAGG | 164 |

Note: The lowercase nucleotide sequence is the vector sequence, and the uppercase nucleotide sequence is the TOT-specific primer sequence.

### 3. Functional Verification of the Taxane C4-C20 Oxetane-forming Enzyme TOT Gene in Tobacco

The constructed expression vector pEAQ-HT-TOT was transferred into *Agrobacterium* GV3101 to obtain the GV3101/pEAQ-HT-TOT transgenic strain. The positive single clone was picked and inoculated into LB medium (including 50 µg/mL kanamycin and 25 µg/mL rifampicin), culturing at 28°C for 24 h. The cultured bacterial liquid was taken in 10 mL LB medium (including 50 µg/mL kanamycin and 25 µg/mL rifampicin) at a ratio of 1:100, culturing at 28°C overnight to an OD₆₀₀ value of 0.8-1.0; adding resuspension MMA (10 mM MES, 10 mM MgCl₂, 150 µM acetosyringone) to the final OD₆₀₀ of 0.8-1.0, and allowing to stand at room temperature for 1-2 h.

The resuspended *Agrobacterium* GV3101 (including the TOT gene) was injected into the back of leaves of 4-6 week-old *Nicotiana benthamiana* by a 1 mL syringe without a needle. After drying under light for 1-2 h, the leaves were transferred to a dark place for incubation for 24 h, then transferring to normal light for incubation. Four days after the injection of *Agrobacterium* GV3101, an aqueous solution of taxadiene-hexanol-hexaacetate (compound (1)) was injected into the leaves injected with *Agrobacterium* GV3101 to incubate for another 24 h. Leaves injected with GFP were used as controls.

The tobacco sample was ground with a grinder, and the sample powder was transferred to a 15 mL centrifuge tube. After freeze-drying, dichloromethane and methanol (DCM/MeOH=1:1) were added, and ultrasonic extraction was performed for 5 min. The sample was concentrated by a vacuum concentrator, and the solvent was removed. After adding 500 µL of methanol, the mixture was fully shaken. After the solid was dissolved, the mixture was centrifuged at 12000 rpm for 15 min at room temperature, and then loaded for detection in LC-MS.

As shown in Fig. 1, TOT can transform compound (1) into two isomers, namely 1-dehydroxybaccatin IV (compound (2)), and baccatin I (compound (3)).

### Example 2: Activity Screening of the Mutants of Taxane C4-C20 Oxetane-forming Enzyme TOT Gene

### 1. Construction of Taxane C4-C20 Oxetane-forming Enzyme TOT Mutant

The recombinant plasmid pEAQ-HT-TOT was used as a template, and point mutation primers were designed (Table 4). After amplifying the plasmid including the TOT gene with high-fidelity enzyme by PCR, a plasmid with an amino acid point mutation was obtained. After ligation, transformation and sequencing, the plasmid was extracted, and the TOT gene including the point mutation was amplified by using a high-fidelity enzyme. The TOT gene was constructed into the tobacco expression vector pEAQ-HT by ClonExpress homologous recombination cloning kit (Ultra One Step Cloning Kit) to obtain the recombinant plasmid pEAQ-HT::TOTsm, which was then transferred into *Agrobacterium* GV3101 to obtain GV3101/pEAQ-HT-TOTsm transgenic *Agrobacterium. Agrobacterium* including pEAQ-HT::TOT1 was used as a control strain.

### 2. Functional Validation of the Taxane C4-C20 Oxetane-forming Enzyme TOT Mutant in Tobacco

A positive single clone of *Agrobacterium* was picked to inoculate into LB medium (including 50 µg/mL kanamycin and 25 µg/mL rifampicin), culturing at 28°C for 24 h. The cultured bacterial solution was taken to add into 10 mL of LB medium (including 50 µg/mL kanamycin and 25 µg/mL rifampicin) at a ratio of 1:100, culturing at 28°C overnight until the OD₆₀₀ value is 0.8-1.0; adding resuspension solution MMA (10 mM MES, 10 mM MgCl₂, 150 µM acetosyringone) to the final OD₆₀₀ of 0.8-1.0, then standing at room temperature for 1-2 h.

The resuspended *Agrobacterium* (including the TOTsm mutant gene) was injected into the underside of leaves of 4-6 week-old *Nicotiana benthamiana* by a 1 mL syringe without a needle. After drying under light for 1-2 h, the leaves were transferred to a dark place for incubation for 24 h, then transferring to normal light for incubation. Four days after the injection of *Agrobacterium,* the aqueous solution of compound (1) was injected into the leaves injected with *Agrobacterium,* and the incubation was continued for 24 h.

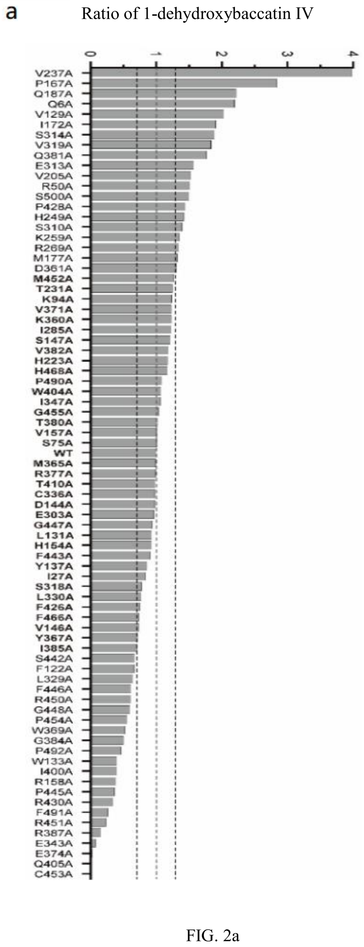

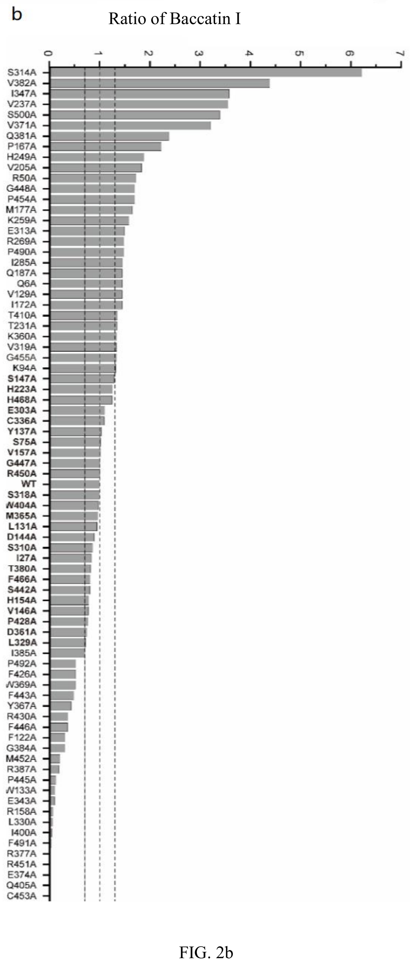

The tobacco sample was ground with a grinder, and the sample powder was transferred to a 15 mL centrifuge tube. After freeze-drying, dichloromethane and methanol (DCM/MeOH=1:1) were added, and ultrasonic extraction was performed for 5 min. The sample was concentrated by a vacuum concentrator, and the solvent was removed, then adding 500 µL of methanol to fully shaken. After the solid was dissolved, the mixture was centrifuged at 12000 rpm for 15 min at room temperature, then loading for detection in LC-MS. Based on the ionic strength, 1-dehydroxybaccatin IV (compound (2)) and baccatin I (compound (3)) produced by different mutants of TOT in tobacco were compared with the wild type (WT), and the fold changes of the resulting ionic strengths are shown in Figs. 2a-2b.

The results of activity comparison show (Figs. 2a-2b):
Different TOT mutants have different effects on their enzyme activity. For the oxetane product, mutants such as V237A, P167A, Q187A, Q6A, V129A, I172A, S314A, V319A, Q381A, E313A, V205A, R50A, S500A, P428A, H249A, S310A, K259A, R269A, M177A, and D361A cause a significant increase in the content of 1-dehydroxybaccatin IV, indicating that optimizing the amino acids at these sites can improve the transformation efficiency of TOT to compound (2), while mutants such as S442A, F122A, L329A, F446A, R450A, G448A, P454A, W369A, G384A, P492A, W133A, I400A, R158A, P445A, R430A, F491A, R451A, R387A, E343A, E374A, Q405A, and C453A cause a decrease in the yield of compound (2) or even no detection, indicating that these sites are crucial for maintaining the activity of TOT.

For the oxirane product, mutants such as S314A, V382A, I347A, V237A, S500A, V371A, Q381A, P167A, H249A, V205A, R50A, G448A, P454A, M177A, K259A, E313A, R269A, P490A, I285A, Q187A, Q6A, V129A, I172A, T410A, T231A, K360A, V319A, G455A, and K94A increase the content of compound (3) produced by TOT, which also indicates that further optimization of these sites improves the activity of TOT, especially the enzymatic efficiency of transforming the C4-C20 double bond to produce a three-membered ring, while mutants such as I385A, P492A, F426A, W369A, F443A, Y367A, R430A, F446A, F122A, G384A, M452A, R387A, P445A, W133A, E343A, R158A, L330A, I400A, F491A, R377A, R451A, E374A, Q405A, and C453A reduce the activity of TOT in catalyzing compound (1) to produce compound (3), or even make TOT lose its catalytic ability.

Therefore, amino acid sites such as V237, P167, Q187, Q6, V129, I172, S314, V319, Q381, E313, V205, R50, S500, P428, H249, S310, K259, R269, M177, D361, V382, I347, V371, G448, P454A, P490, I285, T410, T231, K360, G455, and K94 play an important role in improving the activity of TOT. Further optimization of these sites is likely to obtain highly active mutants, so that substrates including C4-C20 double bonds may be more efficiently transformed into oxetane or oxirane products, and the precursor substances may be more efficiently transformed into paclitaxel, and may be better used for modification of the total synthesis pathway of paclitaxel. However, the amino acid sites I385, S442, F122, L329, F446, R450, G448, P454, W369, G384, P492, W133, I400, R158, P445, R430, F491, R451, R387A, E343, E374, Q405, C453, F426, F443, Y367, M452, L330, and R377 play an important role in maintaining the catalytic activity of TOT, especially the sites E374, Q405, and C453, which can determine whether TOT has catalytic activity. Therefore, these sites need to be paid special attention to in the subsequent optimization, and they should not be easily mutated into alanine or other amino acids that reduce or lose their activity.

### Example 3: Construction of Engineered Strains and Methods for Producing 1-Dehydroxybaccatin IV and Baccatin I

### 1. Construction of Engineered Yeast Strains

The chassis engineering yeast strain was WAT11 (MATa leu2-3, 112trp1-1 canl-100 ura3-1 ade2-1his3-11,15). Primers P3 and P4 were designed, and the recombinant plasmid pEAQ-HT-TOT was used as a template to amplify the TOT gene by PrimeSTAR high-fidelity enzyme PCR. The TOT gene was constructed under the GAL1 promoter in the galactose-inducible yeast expression vector pESC-Leu (including the *Taxus* cytochrome P450 reductase TcCPR gene) by using the ClonExpress homologous recombination cloning kit (Ultra One Step CloningKit) to obtain a recombinant plasmid pESC-Leu::TOT, which was further introduced into the chassis strain WAT11 by the PEG/LiAc method to obtain the engineering strain WAT11/TOT for producing 1-dehydroxybaccatin IV and baccatin I. At the same time, the control engineering strain WAT11/pESC-Leu was obtained by introducing the empty vector pESC-Leu.

The specific primers for the PCR gene are shown in Table 4.

### 2. Method for Producing 1-Dehydroxybaccatin IV and Baccatin I by Fermentation of the Engineered Bacteria

One monoclonal *Saccharomyces cerevisiae* engineered bacteria WAT11/TOT was selected to inoculate into 30 mL of SD-Leu liquid medium including 2% glucose, then culturing overnight at 28°C in a shaker at 250 rpm. Yeast solution was added to 50 mL of YPGA medium in a ratio of 1 OD₆₀₀/100 mL of YPGA medium (10 g of yeast extract, 20 g of peptone, 74 mg of adenine sulfate, and 100 mL of glucose solution (200 g/L), diluting to a final volume of 1 L with water), and the mixture was cultured in a shaker at 28°C and 250 rpm for 30 h. The bacterial cells were collected by centrifugation at 5000×g for 5 min, washing the bacterial cells three times with 50 mL of sterile deionized water, and then resuspending in 50 mL of induction medium YPLA (10 g yeast extract, 20 g peptone, 74 mg adenine sulfate, and 100 mL galactose solution (200 g/L), diluting to a final volume of 1 L with water). Induction culture was performed for the mixture in a shaker at 28°C and 250 rpm for 16 h. 10 mL of the bacterial solution was taken to centrifuge at 5000×g for 5 min, then resuspending in 500 µL YPLA medium. Compound (1) with a final concentration of 50 µM and 4% DMSO were added in the medium to culture for another 24 h, then adding ethyl acetate to perform ultrasonic extraction for 30 min. The mixture was centrifuged at 10000 rpm for 10 min at room temperature. The upper layer of liquid was retained, and the extraction was repeated once to combine the two upper layer liquids for vacuum drying, then reconstituting with methanol for LC-MS detection.

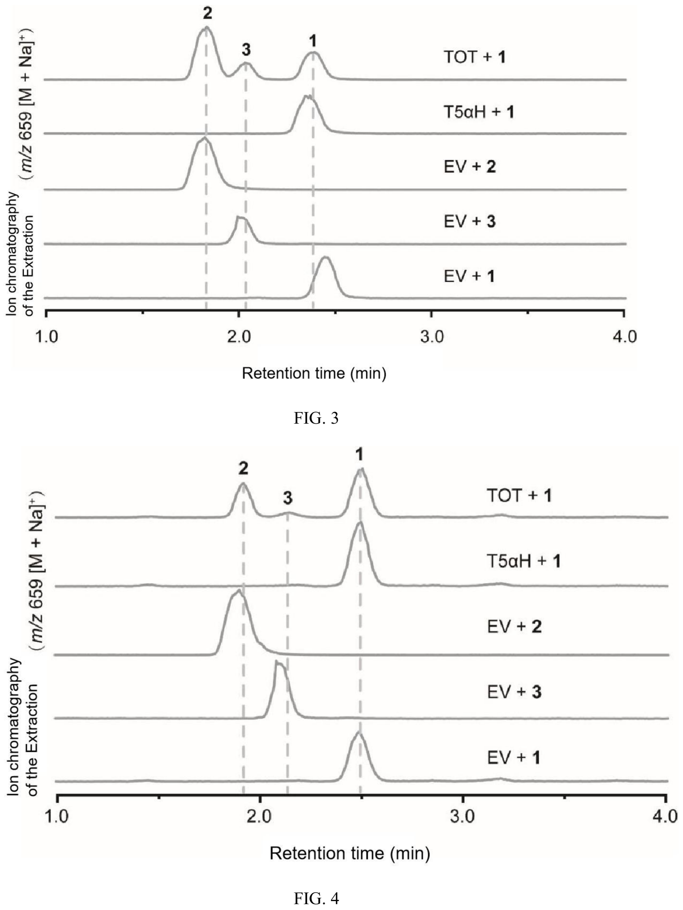

The LC-MS results show (Fig. 3) that, the fermentation of the engineered bacteria produces two products similar to those in the tobacco expression system, whose retention times correspond to 1-dehydroxybaccatin IV and baccatin I, respectively, thus corroborating the results of the tobacco expression system.

### Example 4: Taxane C4-C20 Oxetane-forming Enzyme TOT was Expressed in Insect Cells, and Catalyzed the Formation of Oxetane and Oxirane from the C4-C20 double Bond of Taxane

### 1. Plasmid Construction

The insect cells used are Sf9 cells, which are clones of Sf21 cells developed from the ovarian tissue of the pupal stage of *Spodoptera frugiperda.* The recombinant plasmids PEAQ-HT-TOT and PEAQ-HT-CPR were used as templates, the primers P5 and P6, P7 and P8 were designed respectively, and TOT and CPR (cytochrome P450 reductase) genes were amplified by PimeSTAR high-fidelity enzyme PCR. The two genes were constructed under the polyhedrin promoter of the insect cell expression vector pFastBacl by the ClonExpress homologous recombination cloning kit (Ultra One Step Cloning Kit) to obtain recombinant plasmids pFastBacl-TOT and PEAQ-HT-CPR. The recombinant plasmids were used to further transform the *Escherichia coli* DH10Bac strain including the baculovirus shuttle vector Bacmid and a helper plasmid (used to express the transposition protein required for transposition) to obtain the recombinant plasmids Bacmid-TOT and Bacmid-CPR. At the same time, the control strain Bacmid was obtained by introducing the control vector pFastBac1.

Among the primers used, P5 and P6 were used to amplify TOT, and P7 and P8 were used to amplify CPR. The primer sequences are shown in Table 4.

### 2. Activity Identification of TOT Protein Expressed in Insect Cells

Bacmid-TOT1, Bacmid-CPR and Bacmid were transfected into insect cells Sf9 by linearized polyethyleneimine (PEI) transfection reagent. After 3 days of culture, P1 generation virus was obtained. P1 generation virus was transfected into Sf9 cells to obtain P2 generation virus. The experiment was repeated to obtain P4 generation virus. P4 generation viruses of Bacmid-TOT, Bacmid-CPR and Bacmid were used to infect 50 mL of Sf9 suspension cells, respectively. After culturing at 100 rpm, 28°C for 2 days, the cells were centrifuged at 100 g for 5 min at room temperature, then resuspending with 500 µL of supernatant. Compound (1) with a final concentration of 50 µM and 4% DMSO were added to continuously culture the cells for another 24 h, then adding ethyl acetate to ultrasonically extract the cells for 30 min, and centrifuging the cells at 10,000 rpm for 10 min at room temperature to retain the upper liquid. After repeated the extraction, the two upper liquids were combined for vacuum drying, then reconstituting with methanol for LC-MS detection.

The LC-MS results show (Fig. 4) that, TOT expressed in insect cells also can transform compound (1) into compound (2) and compound (3), whose retention times correspond to 1-dehydroxybaccatin IV and baccatin I respectively, thus corroborating the results of the tobacco and yeast expression systems.

### Example 5: Comparison of the Activities of Taxane C4-C20 Oxetane-forming Enzyme TOT and a Homologous Gene thereof

### 1. Construction of a homologous gene of the taxane C4-C20 oxetane-forming enzyme TOT

Based on the nucleotide sequence of TOT homologous genes (TOT2/3/4), amplification primers P9 and P10, P11 and P12, P13 and P14 were designed. The primers include part of the sequence of tobacco expression vector pEAQ-HT. The cDNA of the leaves of *Taxus chinensis* var. *maire* was used as a template to amplify the TOT2/3/4 genes by PCR. The PCR products were recovered by gel excision, and recombined with the linear pEAQ-HT vector digested with RruI and XhoI. The ClonExpress one-step cloning kit (Vazyme Biotech) was used for cloning and sequencing. The positive recombinant plasmids were named pEAQ-HT-TOT2, pEAQ-HT-TOT3, and pEAQ-HT-TOT4, respectively. The recombinant plasmids were respectively transferred into *Agrobacterium* GV3101 to obtain GV3101/pEAQ-HT-TOT2/3/4 transgenic *Agrobacterium,* and *Agrobacterium* including pEAQ-HT::TOT1 was used as a control strain.

Among the primers used, P9 and P10 were used to amplify TOT2, P11 and P12 were used to amplify TOT3, and P13 and P14 were used to amplify TOT4. The primer sequences are shown in Table 4.

### 2. Comparison of the activities of taxane C4-C20 oxetane-forming enzyme TOT and a homologous gene thereof in tobacco

A positive single clone of *Agrobacterium* was picked to inoculate into LB medium (including 50 µg/mL kanamycin and 25 µg/mL rifampicin), culturing at 28°C for 24 h. The cultured bacterial solution was taken to add into 10 mL of LB medium (including 50 µg/mL kanamycin and 25 µg/mL rifampicin) at a ratio of 1:100, culturing at 28°C overnight until the OD₆₀₀ value is 0.8-1.0; adding resuspension solution MMA (10 mM MES, 10 mM MgCl₂, 150 µM acetosyringone) to the final OD₆₀₀ of 0.8-1.0, then standing at room temperature for 1-2 h.

The resuspended *Agrobacterium* (including the TOT1/2/3/4 genes) was injected into the underside of leaves of 4-6 week-old *Nicotiana benthamiana* by a 1 mL syringe without a needle. After drying under light for 1-2 h, the leaves were transferred to a dark place for incubation for 24 h, then transferring to normal light for incubation. Four days after the injection of *Agrobacterium,* the aqueous solution of compound (1) was injected into the leaves injected with *Agrobacterium,* and the incubation was continued for 24 h.

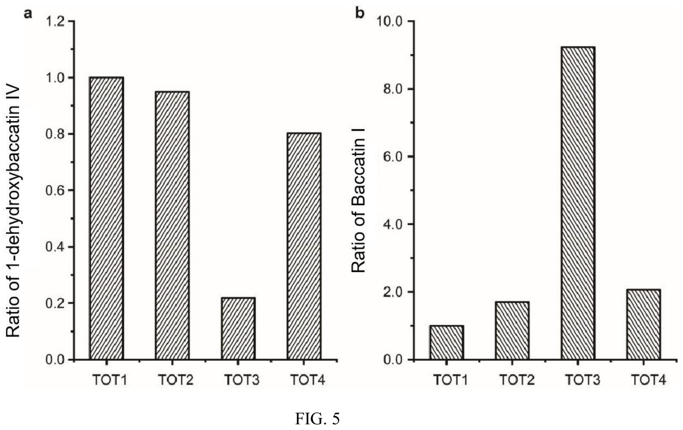

The tobacco sample was ground with a grinder, and the sample powder was transferred to a 15 mL centrifuge tube. After freeze-drying, dichloromethane and methanol (DCM/MeOH=1:1) were added, and ultrasonic extraction was performed for 5 min. The sample was concentrated by a vacuum concentrator, and the solvent was removed, then adding 500 µL of methanol to fully shaken. After the solid was dissolved, the mixture was centrifuged at 12000 rpm for 15 min at room temperature, then loading for detection in LC-MS. Based on the ionic strength, 1-dehydroxybaccatin IV (compound (2)) and baccatin I (compound (3)) produced by different homologous genes of TOT in tobacco were compared with TOT1, and the fold changes of the resulting ionic strengths are shown in Fig. 5.

The results of the activity comparison of the taxane C4-C20 oxetane-forming enzyme TOT and a homologous gene thereof show that, compared with TOT1, the difference in the content of the four-membered ring product compound (2) produced by TOT2, TOT3 and TOT4 was not obvious, while the difference in the content of the three-membered ring product compound (3) was quite obvious, particularly, TOT3 may significantly increase the yield of the three-membered ring product compound (3).

### Example 6: Comparison of the Catalytic Activities of Taxane C4-C20 oxetane-forming Enzyme TOT and a Homologous Gene thereof towards Different Types of Taxanes

### 1. Construction of a homologous gene of the taxane C4-C20 oxetane-forming enzyme TOT

The operations are the same as Example 5.

### 2. Functional characterization of taxane C4-C20 oxetane-forming enzyme TOT and a homologous gene thereof in tobacco

A positive single clone of *Agrobacterium* was picked to inoculate into LB medium (including 50 µg/mL kanamycin and 25 µg/mL rifampicin), culturing at 28°C for 24 h. The cultured bacterial solution was taken to add into 10 mL of LB medium (including 50 µg/mL kanamycin and 25 µg/mL rifampicin) at a ratio of 1:100, culturing at 28°C overnight until the OD₆₀₀ value is 0.8-1.0; adding resuspension solution MMA (10 mM MES, 10 mM MgCl₂, 150 µM acetosyringone) to the final OD₆₀₀ of 0.8-1.0, then standing at room temperature for 1-2 h.

The resuspended *Agrobacterium* (including the TOTsm mutant gene) was injected into the underside of leaves of 4-6 week-old *Nicotiana benthamiana* by a 1 mL syringe without a needle. After drying under light for 1-2 h, the leaves were transferred to a dark place for incubation for 24 h then transferring to normal light for incubation. Four days after the injection of *Agrobacterium,* the aqueous solution of compound (1) or taxadiene-2α,5α,7β,9β,10β,13α-hexanol-5α-acetate (compound (4)) was injected into the leaves injected with *Agrobacterium,* and the incubation was continued for 24 h.

The tobacco sample was ground with a grinder, and the sample powder was transferred to a 15 mL centrifuge tube. After freeze-drying, dichloromethane and methanol (DCM/MeOH=1:1) were added, and ultrasonic extraction was performed for 5 min. The sample was concentrated by a vacuum concentrator, and the solvent was removed, then adding 500 µL of methanol to fully shaken. After the solid was dissolved, the mixture was centrifuged at 12000 rpm for 15 min at room temperature, then loading for detection in LC-MS. The quantitative results of the oxidation products formed by catalyzing compound (1) or compound (4) with tobacco expressed TOT1 or TOT3 are shown in a and b of Fig. 6; and the results of the oxidation products formed by catalyzing compound (1), compound (5), compound (6) and compound (7) with tobacco expressed TOT1 are shown in c, d and e of Fig. 6.

The results show that, both TOT1 and TOT3 may catalyze compound (1) and compound (4) to produce oxidation products. Overall, TOT1 and TOT3 have higher transformation efficiency in catalyzing compound (1) to produce oxidation products (m/z 659[M+Na]⁺), while their catalytic efficiency in catalyzing compound (4) to form oxidation products (m/z 449[M+ Na]⁺) was lower. It was also found that, TOT3 have higher catalytic efficiency for both compounds (1) and (4) than TOT1.

In addition, TOT1 can catalyze compound (1) and compound (5) to form two monooxidized products including oxetane and ethylene oxide, respectively. When the compound lacks the C5 acetyl group, TOT1 can also catalyze the corresponding compound, for example, TOT1 can catalyze compound (6) or compound (7) to form an oxidized product.

In summary, the application provides a taxane C4-C20 oxetane-forming enzyme TOT which catalyzes the carbon-carbon double bond at the C4-C20 position of the taxane to form oxetane and oxirane, laying a foundation for further comprehensive analysis of the paclitaxel biosynthetic pathway. The application further provides a yeast engineering strain WAT11/TOT and insect-expressed TOT, and finds that amino acids I385, S442, F122, L329, F446, R450, G448, P454, W369, G384, P492, W133, I400, R158, P445, R430, F491, R451, R387A, E343, E374, Q405, C453, F426, F443, Y367, M452, L330 and R377 are required to maintain the activity of TOT; while the amino acid sites such as V237, P167, Q187, Q6, V129, I172, S314, V319, Q381, E313, V205, R50, S500, P428, H249, S310, K259, R269, M177, D361, V382, I347, V371, G448, P454A, P490, I285, T410, T231, K360, G455 and K94 play an important role in improving the transformation efficiency of substrates to four-membered ring products 1-dehydroxybaccatin IV, baccatinIII or paclitaxel, and can be better applied to the transformation and optimization of the synthetic pathways of paclitaxel and other related compounds with oxetane ring groups or oxirane ring groups, and have significant economic and ecological value.

The above described are only preferred embodiments of the application. It should be pointed out that for ordinary technicians in this technical field, several improvements and modifications can be made without departing from the principle of the application. These improvements and modifications should also be regarded as falling in the protection scope of the application.

## Claims

1. A bioenzyme, **characterized in that** it comprises at least one of the following:
a1) the amino acid sequence represented by SEQ ID NO: 25;
a2) a fusion protein obtained by connecting a tag to N-terminus and/or C-terminus of the amino acid sequence represented by SEQ ID NO: 25;
a3) a protein obtained by substituting and/or deleting and/or adding one or more amino acid residues in the amino acid sequence represented by SEQ ID NO: 25 and having the same function;
a4) a protein having at least 60% homology with the amino acid sequence represented by SEQ ID NO: 25 and having the same function.

2. The bioenzyme according to claim 1, **characterized in that** the protein of a3) is a mutant of the amino acid sequence of a1), and the mutant comprises at least one of: V237A, P167A, Q187A, Q6A, V129A, I172A, S314A, V319A, Q381A, E313A, V205A, R50A, S500A, P428A, H249A, S310A, K259A, R269A, M177A, D361A, V382A, I347A, V371A, G4481, P454AA, P490A, I285A, T410A, T231A, K360A, G455A, or K94A mutant.

3. The bioenzyme according to claim 1, **characterized in** a4), the amino acid sequence having at least 60% homology with the amino acid sequence represented by SEQ ID NO: 25 and having the same function comprises at least one of the amino acid sequences represented by SEQ ID NOs: 26-48.

4. A nucleic acid molecule encoding the bioenzyme according to any one of claims 1-3, **characterized in that** it comprises at least one of the following sequences:
b1) the nucleotide sequence represented by SEQ ID NO: 1;
b2) a complementary sequence, degenerate sequence or homologous sequence of the nucleotide sequence represented by SEQ ID NO: 1, wherein the homologous sequence is a nucleotide sequence having at least 75% homology with the nucleotide sequence represented by SEQ ID NO: 1;
b3) a nucleotide sequence hybridizing with the nucleotide sequence of b1) or b2) under stringent conditions and being capable of encoding a protein with the same function.

5. The nucleic acid molecule according to claim 4, **characterized in** b2), the homologous sequence comprises at least one nucleotide sequences represented by SEQ ID NOs: 2-24.

6. A biomaterial, **characterized in that** the biomaterial is any one of the following c1) to c4):
c1) an expression cassette comprising the nucleic acid molecule according to claim 4 or 5;
c2) a vector comprising the nucleic acid molecule according to claim 4 or 5, or comprising the expression cassette of c1);
c3) a recombinant microorganism comprising at least one of: the above gene, the expression cassette of cl), and the recombinant vector of c2);
c4) a recombinant plant cell and/or a recombinant animal cell comprising at least one of: the above gene, the expression cassette of c1), or the recombinant vector of c2).

7. The biomaterial according to claim 6, **characterized in that** the microorganism comprises bacteria and/or fungi;
the plant cell comprises at least one of: tobacco cell, *Pseudotaxus chienii* cell, *Artemisia caruifolia* cell, *Arabidopsis thaliana* cell, *Physcomitrium patens* cell, *Marchantia polymorpha* cell, tomato cell, ginseng cell, cotton cell, sugarcane cell, potato cell, corn cell, wheat cell, rice cell, radish cell, lettuce cell, or algae cell; and
the animal cell comprises at least one of: insect cell, mammalian cell, nematode cell, or fish cell.

8. A method for producing a host cell, **characterized in that** the method comprises transforming the host cell with at least one of: the nucleic acid molecule according to claim 4 or 5, and the expression cassette or vector of the biomaterial according to claim 6 or 7.

9. A method for producing a plant or a plant cell, **characterized in that** the method comprises transforming the plant or plant cell with at least one of: the nucleic acid molecule according to claim 4 or 5, or the expression cassette or vector in the biomaterial according to claim 6 or 7.

10. A method for producing the bioenzyme according to any one of claims 1-3, **characterized in that** a host cell is transformed with at least one of: the nucleic acid molecule according to claim 4 or 5, or the expression cassette or vector in the biomaterial according to claim 6 or 7, so that the host cell expresses the bioenzyme.

11. Use of the bioenzyme according to any one of claims 1-3, the nucleic acid molecule according to claim 4 or 5, or the biomaterial according to claim 6 or 7 in the synthesis of oxygen-containing heterocyclic compounds, **characterized in that** the oxygen-containing heterocyclic compound comprises a compound having an oxetane ring group and/or a compound having an oxirane ring group.

12. The use according to claim 11, **characterized in that** the compound having an oxetane ring group comprises a taxane compound, and/or an intermediate thereof;
preferably, the taxane compound comprises at least one of: paclitaxel and a derivative thereof, docetaxel and a derivative thereof, or cabazitaxel and a derivative thereof;
preferably, the intermediate of taxane compound comprises at least one of: 1-dehydroxybaccatin IV and a derivative thereof, baccatin III and a derivative thereof, or 10-deacetylbaccatin III and a derivative thereof.

13. The use according to claim 11 or 12, **characterized in that** the compound having oxirane ring group comprises at least one of: baccatin I and a derivative thereof, Taxumairol C and a derivative thereof, and Taxuchin A and a derivative thereof.

14. A method for synthesizing an oxygen-containing heterocyclic compound in vivo or in vitro, **characterized in that** a taxane compound comprising 5α-acetoxy-4,20-alkenyl or a taxane compound comprising 5α-hydroxy-4,20-alkenyl is used as a substrate to synthesize the oxygen-containing heterocyclic compound under the catalytic action of the bioenzyme according to any one of claims 1-3;
the oxygen-containing heterocyclic compound comprises: a compound having an oxetane ring group, and/or a compound having an oxirane ring group.

15. The method according to claim 14, **characterized in that** the substrate comprises at least one of: taxadiene-hexaol-hexaacetate, taxadiene-2α,5α,7β,9β,10β,13α-hexaol-5α-acetate, 5α-acetate-Taxuspine F, taxadiene-hexaol-tetraacetate, or Taxuspine F.

16. The method according to claim 14 or 15, **characterized in that** the in vivo comprises in a microorganism, an algal cell, a plant cell and/or an animal cell.

17. The method according to any one of claims 14-16, **characterized in that** synthesizing the oxygen-containing heterocyclic compound in vivo comprises: allowing the nucleic acid molecule according to claim 3 to express the bioenzyme in a microbial cell, algal cell, plant cell, and/or animal cell; using the taxane compound comprising 5α-acetoxy-4,20- alkenyl or the taxane compound comprising 5α-hydroxy-4,20-alkenyl as a substrate to synthesize the oxygen-containing heterocyclic compound under the catalytic action of the bioenzyme.

18. A plant or a plant part thereof, **characterized in that** the plant is one of the following plants:
(dl) a transgenic plant comprising the nucleic acid molecule according to claim 4 or 5;
(d2) a plant formed by growth of the plant cell according to claim 6 or 7;
(d3) a plant produced by the method according to claim 9;
(d4) an offspring formed by self-pollination of any plant of (dl) to (d3), as well as a plant formed by growth of the offspring;
(d5) an offspring formed by hybridization of any plant of (dl) to (d3) with other varieties, as well as a plant formed by growth of the offspring;
the above plant part comprises a root, stem, leaf, flower, fruit, pollen or seed.

19. A method of manufacturing a commercial product, **characterized in that** the method comprises: obtaining the plant or plant part thereof according to claim 18, and manufacturing the commercial product from the plant or plant part thereof;
preferably, the commercial product is selected from the group consisting of: a crude extract, an active pharmaceutical ingredient, and/or a pharmaceutical formulation comprising at least one of baccatin III and a derivative thereof, paclitaxel and a derivative thereof, taxusin and a derivative thereof, docetaxel and a derivative thereof, and cabataside and a derivative thereof.
